# FASCICULE DE BREVET EUROPEEN

(11) **EP 3 071 693 B1**
(45) Date de publication et mention de la délivrance du brevet: **02.05.2018**
(21) Numéro de dépôt: 14821724.3
(22) Date de dépôt: 21.11.2014
(51) Int. Cl.: C12N 9/42, D21C 5/00

(54) **VARIANTS D'ENDOGLUCANASES A ACTIVITE AMELIOREE ET LEURS UTILISATIONS**
ENDOGLUCANASE-VARIANTEN MIT VERBESSERTER WIRKUNG UND VERWENDUNGEN DAVON
ENDOGLUCANASE VARIANTS HAVING IMPROVED ACTIVITY, AND USES OF SAME

(30) Priorité: 22.11.2013 FR 1361511
(43) Date de publication de la demande: 28.09.2016
(73) Titulaire: IFP Energies nouvelles, 92500 Rueil-Malmaison (FR); Proteus, 91160 Longjumeau (FR); Centre National de la Recherche Scientifique (C.N.R.S.), 75016 Paris (FR)
(72) Inventeur: MARGEOT, Antoine, F-75018 Paris (FR); BLANQUET, Senta, F-78112 Fourqueux (FR); PERSILLON, Cécile, F-30000 Nimes (FR); AYRINHAC, Céline, F-30350 Domessargues (FR); ULLMANN, Christophe, F-30000 Nimes (FR); BONZOM, Olivier, F-30000 Nimes (FR); FORT, Sébastien, F-38410 Vaulnaveys-le-Haut (FR); ARMAND, Sylvie, F-38100 Grenoble (FR); LENON, Marine, F-38360 Sassenage (FR); PETIT, Maud, F-59700 Marcq en Baroeul (FR)
(74) Mandataire: Cabinet Plasseraud
(86) Numéro de dépôt international: PCT/FR2014/052985
(87) Numéro de publication internationale: WO 2015/075392

(56) Documents cités:
- WO-A1-2013/096244
- WO-A2-2008/088724
- WO-A2-2011/153516
- WO-A2-2012/036810

### SEQUENCE LISTING

<110> IFPEN
<120> VARIANTS D'ENDOGLUCANASES A ACTIVITE AMELIOREE ET LEURS UTILISATIONS
<130> BFF130
<160> 38
<170> PatentIn version 3.5
<210> 1
   <211> 1257
   <212> DNA
   <213> Trichoderma reesei
<400> 1
   atgaacaagt ccgtggctcc attgctgctt gcagcgtcca tactatatgg cggcgccgct      60
   gcacagcaga ctgtctgggg ccagtgtgga ggtattggtt ggagcggacc tacgaattgt     120
   gctcctggct cagcttgttc gaccctcaat ccttattatg cgcaatgtat tccgggagcc     180
   actactatca ccacttcgac ccggccacca tccggtccaa ccaccaccac cagggctacc     240
   tcaacaagct catcaactcc acccacgagc tctggggtcc gatttgccgg cgttaacatc     300
   gcgggttttg actttggctg taccacagat ggcacttgcg ttacctcgaa ggtttatcct     360
   ccgttgaaga acttcaccgg ctcaaacaac taccccgatg gcatcggcca gatgcagcac     420
   ttcgtcaacg acgacgggat gactattttc cgcttacctg tcggatggca gtacctcgtc     480
   aacaacaatt tgggcggcaa tcttgattcc acgagcattt ccaagtatga tcagcttgtt     540
   caggggtgcc tgtctctggg cgcatactgc atcgtcgaca tccacaatta tgctcgatgg     600
   aacggtggga tcattggtca gggcggccct actaatgctc aattcacgag cctttggtcg     660
   cagttggcat caaagtacgc atctcagtcg agggtgtggt tcggcatcat gaatgagccc     720
   cacgacgtga acatcaacac ctgggctgcc acggtccaag aggttgtaac cgcaatccgc     780
   aacgctggtg ctacgtcgca attcatctct ttgcctggaa atgattggca atctgctggg     840
   gctttcatat ccgatggcag tgcagccgcc ctgtctcaag tcacgaaccc ggatgggtca     900
   acaacgaatc tgatttttga cgtgcacaaa tacttggact cagacaactc cggtactcac     960
   gccgaatgta ctacaaataa cattgacggc gccttttctc cgcttgccac ttggctccga    1020
   cagaacaatc gccaggctat cctgacagaa accggtggtg gcaacgttca gtcctgcata    1080
   caagacatgt gccagcaaat ccaatatctc aaccagaact cagatgtcta tcttggctat    1140
   gttggttggg gtgccggatc atttgatagc acgtatgtcc tgacggaaac accgactggc    1200
   agtggtaact catggacgga cacatccttg gtcagctcgt gtctagcaag aaagtag       1257
<210> 2
   <211> 418
   <212> PRT
   <213> Trichoderma reesei
<400> 2
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305                 310                 315                 320
   Ala Glu Cys Thr Thr Asn Asn Ile Asp Gly Ala Phe Ser Pro Leu Ala
                   325                 330                 335
   Thr Trp Leu Arg Gln Asn Asn Arg Gln Ala Ile Leu Thr Glu Thr Gly
               340                 345                 350
   Gly Gly Asn Val Gln Ser Cys Ile Gln Asp Met Cys Gln Gln Ile Gln
           355                 360                 365
   Tyr Leu Asn Gln Asn Ser Asp Val Tyr Leu Gly Tyr Val Gly Trp Gly
       370                 375                 380
   Ala Gly Ser Phe Asp Ser Thr Tyr Val Leu Thr Glu Thr Pro Thr Gly
   385                 390                 395                 400
   Ser Gly Asn Ser Trp Thr Asp Thr Ser Leu Val Ser Ser Cys Leu Ala
                   405                 410                 415
   Arg Lys
<210> 3
   <211> 1257
   <212> DNA
   <213> Artificial Sequence
<220>
   <223> 37D12
<400> 3
   atgaacaagt ccgtggctcc attgctgctt gcagcgtcca tactatatgg cggcgccgct 60
   gcacagcaga ctgtctgggg ccagtgtgga ggtattggtt ggagcggacc tacgaattgt 120
   gctcctggct cagcttgttc gaccctcaat ccttattatg cgcaatgtat tccgggagcc 180
   actactatca ccacttcgac ccggccacca tccggtccaa ccaccaccac cagggctacc 240
   tcaacaagct catcaactcc acccacgagc tctggggtcc gatttgccgg cgttaacatc 300
   gcgggttttg actttggctg taccacagat ggcacttgcg ttacctcgaa ggtttatcct 360
   ccgttgaaga acttcaccgg ctcaaacaac taccccgatg gcatcggcca gatgcagcac 420
   ttcgtcaacg acgacgggat gactattttc cgcttacctg tcggatggca gtacctcgtc 480
   aacaacaatt tgggcggcaa tcttgattcc acgagcattt ccaagtatga tcagcttgtt 540
   caggggtgcc tgtctctggg cgcatactgc atcgtcgaca tccacaatta tgctcgatgg 600
   aacggtggga tcattggtca gggcggccct actaatgctc aattcacgag cctttggtcg 660
   cagttggcat caaagtacgc atctcagtcg agggtgtggt tcggcatcat gaatgagccc 720
   cacgacgtga acatcaacac ctgggctgcc acggtccaag aggttgtaac cgcaatccgc 780
   aacgctggtg ctacgtcgca attcatctct ttgcctggaa atgattggca atctgctggg 840
   gctttcatat ccgatggcag tgcagccgcc ctgtctcaag tcacgaaccc ggatgggtca 900
   acaacgaatc tgatttttga cgtgcacaaa tacttggact cagacaactc cggtactcac 960
   gccgaatgtg taacaaataa cattgacgcc gcctttgcac cgcttgccac ttggctccga 1020
   gcaaacggtc gccaggctat cctgagcgaa accggtggtg gcaacaccgc gtcctgccag 1080
   acatatttgt gccagcaagt tgcttatctc aacgcaaact cagatgtcta tcttggctat 1140
   attggttggt ctgccggatc atttgatagc acgtatattc tgacggaaac accgaatggc 1200
   agtggtaact catggacgga cacatccttg gtcagctcgt gtctagcaag aaagtag 1257
<210> 4
   <211> 418
   <212> PRT
   <213> Artificial Sequence
<220>
   <223> 37D12
<400> 4
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
   50 55 60
   Thr Ser Thr Arg Pro Pro Ser Gly Pro Thr Thr Thr Thr Arg Ala Thr
   65 70 75 80
   Ser Thr Ser Ser Ser Thr Pro Pro Thr Ser Ser Gly Val Arg Phe Ala
   85 90 95
   Gly Val Asn Ile Ala Gly Phe Asp Phe Gly Cys Thr Thr Asp Gly Thr
   100 105 110
   Cys Val Thr Ser Lys Val Tyr Pro Pro Leu Lys Asn Phe Thr Gly Ser
   115 120 125
   Asn Asn Tyr Pro Asp Gly Ile Gly Gln Met Gln His Phe Val Asn Asp
   130 135 140
   Asp Gly Met Thr Ile Phe Arg Leu Pro Val Gly Trp Gln Tyr Leu Val
   145 150 155 160
   Asn Asn Asn Leu Gly Gly Asn Leu Asp Ser Thr Ser Ile Ser Lys Tyr
   165 170 175
   Asp Gln Leu Val Gln Gly Cys Leu Ser Leu Gly Ala Tyr Cys Ile Val
   180 185 190
   Asp Ile His Asn Tyr Ala Arg Trp Asn Gly Gly Ile Ile Gly Gln Gly
   195 200 205
   Gly Pro Thr Asn Ala Gln Phe Thr Ser Leu Trp Ser Gln Leu Ala Ser
   210 215 220
   Lys Tyr Ala Ser Gln Ser Arg Val Trp Phe Gly Ile Met Asn Glu Pro
   225 230 235 240
   His Asp Val Asn Ile Asn Thr Trp Ala Ala Thr Val Gln Glu Val Val
   245 250 255
   Thr Ala Ile Arg Asn Ala Gly Ala Thr Ser Gln Phe Ile Ser Leu Pro
   260 265 270
   Gly Asn Asp Trp Gln Ser Ala Gly Ala Phe Ile Ser Asp Gly Ser Ala
   275 280 285
   Ala Ala Leu Ser Gln Val Thr Asn Pro Asp Gly Ser Thr Thr Asn Leu
   290 295 300
   Ile Phe Asp Val His Lys Tyr Leu Asp Ser Asp Asn Ser Gly Thr His
   305                 310                 315                 320
   Ala Glu Cys Val Thr Asn Asn Ile Asp Ala Ala Phe Ala Pro Leu Ala
                   325                 330                 335
   Thr Trp Leu Arg Ala Asn Gly Arg Gln Ala Ile Leu Ser Glu Thr Gly
               340                 345                 350
   Gly Gly Asn Thr Ala Ser Cys Gln Thr Tyr Leu Cys Gln Gln Val Ala
           355                 360                 365
   Tyr Leu Asn Ala Asn Ser Asp Val Tyr Leu Gly Tyr Ile Gly Trp Ser
       370                 375                 380
   Ala Gly Ser Phe Asp Ser Thr Tyr Ile Leu Thr Glu Thr Pro Asn Gly
   385                 390                 395                 400
   Ser Gly Asn Ser Trp Thr Asp Thr Ser Leu Val Ser Ser Cys Leu Ala
                   405                 410                 415
   Arg Lys
<210> 5
   <211> 1260
   <212> DNA
   <213> Artificial Sequence
<220>
   <223> 45A7
<400> 5
   atgaacaagt ccgtggctcc attgctgctt gcagcgtcca tactatatgg cggcgccgct 60
   gcacagcaga ctgtctgggg ccagtgtgga ggtattggtt ggagcggacc tacgaattgt 120
   gctcctggct cagcttgttc gaccctcaat ccttattatg cgcaatgtat tccgggagcc 180
   actactatca ccacttcgac ccggccacca tccggtccaa ccaccaccac cagggctacc 240
   tcaacaagct catcaactcc acccacgagc tctggggtcc gatttgccgg cgttaacatc 300
   gcgggttttg actttggctg taccacagat ggcacttgcg ttacctcgaa ggtttatcct 360
   ccgttgaaga acttcaccgg ctcaaacaac taccccgatg gcatcggcca gatgcagcac 420
   ttcgtcaacg acgacgggat gactattttc cgcttacctg tcggatggca gtacctcgtc 480
   aacaacaatt tgggcggcaa tcttgattcc acgagcattt ccaagtatga tcagcttgtt 540
   caggggtgcc tgtctctggg cgcatactgc atcgtcgaca tccacaatta tgctcgatgg 600
   aacggtggga tcattggtca gggcggccct actaatgctc aattcacgag cctttggtcg 660
   cagttggcat caaagtacgc atctcagtcg agggtgtggt tcggcatcat gaatgagccc 720
   cacgacgtga acatcaacac ctgggctgcc acggtccaag aggttgtaac cgcaatccgc 780
   aacgctggtg ctacgtcgca attcatctct ttgcctggaa atgattggca atctgctggg 840
   gctttcatat ccgatggcag tgcagccgcc ctgtctcaag tcacgaaccc ggatgggtca 900
   acaacgaatc tgatttttga cgtgcacaaa tacttggact cagacaactc cggtactcac 960
   gccgaatgtg taacaaataa cattgacgcc gcctttgcac cgcttgccac ttggctccga 1020
   gcaaacggac gccaggctat cctgacagaa accggtggtg gcaacaccgc gtcctgccag 1080
   acatatttgt gccagcaagt tgcttatctc aacgcaaact cagatgtcta tcttggctat 1140
   attggttggt ctgccggatc atttgatagc acgtatattc tgacggaaac accgaatggc 1200
   agtggttctt caatgacgga ccaagcgttg gtcgcggctt gtctaactag aacatcgtag 1260
<210> 6
   <211> 419
   <212> PRT
   <213> Artificial Sequence
<220>
   <223> 45A7
<400> 6
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305                 310                 315                 320
   Ala Glu Cys Val Thr Asn Asn Ile Asp Ala Ala Phe Ala Pro Leu Ala
                   325                 330                 335
   Thr Trp Leu Arg Ala Asn Gly Arg Gln Ala Ile Leu Thr Glu Thr Gly
               340                 345                 350
   Gly Gly Asn Thr Ala Ser Cys Gln Thr Tyr Leu Cys Gln Gln Val Ala
           355                 360                 365
   Tyr Leu Asn Ala Asn Ser Asp Val Tyr Leu Gly Tyr Ile Gly Trp Ser
       370                 375                 380
   Ala Gly Ser Phe Asp Ser Thr Tyr Ile Leu Thr Glu Thr Pro Asn Gly
   385                 390                 395                 400
   Ser Gly Ser Ser Met Thr Asp Gln Ala Leu Val Ala Ala Cys Leu Thr
                   405                 410                 415
   Arg Thr Ser
<210> 7
   <211> 1257
   <212> DNA
   <213> Artificial Sequence
<220>
   <223> 46H1
<400> 7
   atgaacaagt ccgtggctcc attgctgctt gcagcgtcca tactatatgg cggcgccgct      60
   gcacagcaga ctgtctgggg ccagtgtgga ggtattggtt ggagcggacc tacgaattgt     120
   gctcctggct cagcttgttc gaccctcaat ccttattatg cgcaatgtat tccgggagcc     180
   actactatca ccacttcgac ccggccacca tccggtccaa ccaccaccac cagggctacc     240
   tcaacaagct catcaactcc acccacgagc tctggggtcc gatttgccgg cgttaacatc     300
   gcgggttttg actttggctg taccacagat ggcacttgcg ttacctcgaa ggtttatcct     360
   ccgttgaaga acttcaccgg ctcaaacaac taccccgatg gcatcggcca gatgcagcac     420
   ttcgtcaacg acgacgggat gactattttc cgcttacctg tcggatggca gtacctcgtc     480
   aacaacaatt tgggcggcaa tcttgattcc acgagcattt ccaagtatga tcagcttgtt     540
   caggggtgcc tgtctctggg cgcatactgc atcgtcgaca tccacaatta tgctcgatgg     600
   aacggtggga tcattggtca gggcggccct actaatgctc aattcacgag cctttggtcg     660
   cagttggcat caaagtacgc atctcagtcg agggtgtggt tcggcatcat gaatgagccc     720
   cacgacgtga acatcaacac ctgggctgcc acggtccaag aggttgtaac cgcaatccgc     780
   aacgctggtg ctacgtcgca attcatctct ttgcctggaa atgattggca atctgctggg     840
   gctttcataa ccgatggcag tgcagccgcc ctgtctcaag tcacgaaccc ggatgggtca     900
   acaacgaatc tgatttttga cgtgcacaaa tacttggact cagacaactc cggtactcac     960
   gccgaatgtg tgacaaataa cattgacacc gcctttgcac cgcttgccac ttggctccga    1020
   cagaacaatc gccaggctat cctgacagaa accggtggtg gcaacgttca gtcctgcata    1080
   caagacatgt gccagcaaat ccaatatctc aaccagaact cagatgtcta tcttggctat    1140
   gttggttggg gtgccggatc atttgatagc acgtatgtcc tgacggaaac accgactggc    1200
   agtggtaact catggacgga cacatccttg gtcagctcgt gtctagcaag aaagtag       1257
<210> 8
   <211> 418
   <212> PRT
   <213> Artificial Sequence
<220>
   <223> 46H1
<400> 8
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
   210 215 220
   Lys Tyr Ala Ser Gln Ser Arg Val Trp Phe Gly Ile Met Asn Glu Pro
   225 230 235 240
   His Asp Val Asn Ile Asn Thr Trp Ala Ala Thr Val Gln Glu Val Val
   245 250 255
   Thr Ala Ile Arg Asn Ala Gly Ala Thr Ser Gln Phe Ile Ser Leu Pro
   260 265 270
   Gly Asn Asp Trp Gln Ser Ala Gly Ala Phe Ile Thr Asp Gly Ser Ala
   275 280 285
   Ala Ala Leu Ser Gln Val Thr Asn Pro Asp Gly Ser Thr Thr Asn Leu
   290 295 300
   Ile Phe Asp Val His Lys Tyr Leu Asp Ser Asp Asn Ser Gly Thr His
   305 310 315 320
   Ala Glu Cys Val Thr Asn Asn Ile Asp Thr Ala Phe Ala Pro Leu Ala
   325 330 335
   Thr Trp Leu Arg Gln Asn Asn Arg Gln Ala Ile Leu Thr Glu Thr Gly
   340 345 350
   Gly Gly Asn Val Gln Ser Cys Ile Gln Asp Met Cys Gln Gln Ile Gln
   355 360 365
   Tyr Leu Asn Gln Asn Ser Asp Val Tyr Leu Gly Tyr Val Gly Trp Gly
   370 375 380
   Ala Gly Ser Phe Asp Ser Thr Tyr Val Leu Thr Glu Thr Pro Thr Gly
   385 390 395 400
   Ser Gly Asn Ser Trp Thr Asp Thr Ser Leu Val Ser Ser Cys Leu Ala
   405 410 415
   Arg Lys
<210> 9
   <211> 1257
   <212> DNA
   <213> Artificial Sequence
<220>
   <223> 50F10
<400> 9
   atgaacaagt ccgtggctcc attgctgctt gcagcgtcca tactatatgg cggcgccgct      60
   gcacagcaga ctgtctgggg ccagtgtgga ggtattggtt ggagcggacc tacgaattgt     120
   gctcctggct cagcttgttc gaccctcaat ccttattatg cgcaatgtat tccgggagcc     180
   actactatca ccacttcgac ccggccacca tccggtccaa ccaccaccac cagggctacc     240
   tcaacaagct catcaactcc acccacgagc tctggggtcc gatttgccgg cgttaacatc     300
   gcgggttttg actttggctg taccacagat ggcacttgcg ttacctcgaa ggtttatcct     360
   ccgttgaaga acttcaccgg ctcaaacaac taccccgatg gcatcggcca gatgcagcac     420
   ttcgtcaacg acgacgggat gactattttc cgcttacctg tcggatggca gtacctcgtc     480
   aacaacaatt tgggcggcaa tcttgattcc acgagcattt ccaagtatga tcagcttgtt     540
   caggggtgcc tgtctctggg cgcatactgc atcgtcgaca tccacaatta tgctcgatgg     600
   aacggtggga tcattggtca gggcggccct actaatgctc aattcacgag cctttggtcg     660
   cagttggcat caaagtacgc atctcagtcg agggtgtggt tcggcatcat gaatgagccc     720
   cacgacgtga acatcaacac ctgggctgcc acggtccaag aggttgtaac cgcaatccgc     780
   aacgctggtg ctacgtcgca attcatctct ttgcctggaa atgattggca atctgctggg     840
   gctttcatat ccgatggcag tgcagccgcc ctgtctcaag tcacgaaccc ggatgggtca     900
   acaacgaatc tgatttttga cgtgcacaaa tacttggact cagacaactc cggtactcac     960
   gccgaatgta ctacaaataa cattgacggc gccttttctc cgcttgccac ttggctccga    1020
   cagaacaatc gccaggctat cctgacagaa accggtggtg gcaacgttca gtcctgccag    1080
   acatatttgt gccagcaagt tgcttatctc aacgcaaact cagatgtcta tcttggctat    1140
   attggttggt ctgccggatc atttgatagc acgtatgtcc tgacggaaac accgactggc    1200
   agtggtaact catggacgga cacatccttg gtcagctcgt gtctagcaag aaagtag       1257
<210> 10
   <211> 418
   <212> PRT
   <213> Artificial Sequence
<220>
   <223> 50F10
<400> 10
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305                 310                 315                 320
   Ala Glu Cys Thr Thr Asn Asn Ile Asp Gly Ala Phe Ser Pro Leu Ala
                   325                 330                 335
   Thr Trp Leu Arg Gln Asn Asn Arg Gln Ala Ile Leu Thr Glu Thr Gly
               340                 345                 350
   Gly Gly Asn Val Gln Ser Cys Gln Thr Tyr Leu Cys Gln Gln Val Ala
           355                 360                 365
   Tyr Leu Asn Ala Asn Ser Asp Val Tyr Leu Gly Tyr Ile Gly Trp Ser
       370                 375                 380
   Ala Gly Ser Phe Asp Ser Thr Tyr Val Leu Thr Glu Thr Pro Thr Gly
   385                 390                 395                 400
   Ser Gly Asn Ser Trp Thr Asp Thr Ser Leu Val Ser Ser Cys Leu Ala
                   405                 410                 415
   Arg Lys
<210> 11
   <211> 1257
   <212> DNA
   <213> Artificial Sequence
<220>
   <223> 108G5
<400> 11
   atgaacaagt ccgtggctcc attgctgctt gcagcgtcca tactatatgg cggcgccgct      60
   gcacagcaga ctgtctgggg ccagtgtgga ggtattggtt ggagcggacc tacgaattgt     120
   gctcctggct cagcttgttc gaccctcaat ccttattatg cgcaatgtat tccgggagcc     180
   actactatca ccacttcgac ccggccacca tccggtccaa ccaccaccac cagggctacc     240
   tcaacaagct catcaactcc acccacgagc tctggggtcc gatttgccgg cgttaacatc     300
   gcgggttttg actttggctg taccacagat ggcacttgcg ttacctcgaa ggtttatcct     360
   ccgttgaaga acttcaccgg ctcaaacaac taccccgatg gcatcggcca gatgcagcac     420
   ttcgtcaacg acgacgggat gactattttc cgcttacctg tcggatggca gtacctcgtc     480
   aacaacaatt tgggcggcaa tcttgattcc acgagcattt ccaagtatga tcagcttgtt     540
   caggggtgcc tgtctctggg cgcatactgc atcgtcgaca tccacaatta tgctcgatgg     600
   aacggtggga tcattggtca gggcggccct actaatgctc aattcacgag cctttggtcg     660
   cagttggcat caaagtacgc atctcagtcg agggtgtggt tcggcatcat gaatgagccc     720
   cacgacgtga acatcaacac ctgggctgcc acggtccaag aggttgtaac cgcaatccgc     780
   aacgctggtg ctacgtcgca attcatctct ttgcctggaa atgattggca atctgctggg     840
   gctttcatat ccgatggcag tgcagccgcc ctgtctcaag tcacgaaccc ggatgggtca     900
   acaacgaatc tgatttttga cgtgcacaaa tacttggact cagacaactc cggtactcac     960
   gccgaatgta ctacaaataa cattgacggc gccttttctc cgcttgccac ttggctccga    1020
   gcaaacggtc gccaggctat cctgacagaa accggtggtg gcaacgttca gtcctgcata    1080
   caagacatgt gccagcaaat ccaatatctc aaccagaact cagatgtcta tcttggctat    1140
   gttggttggg gtgccggatc atttgatagc acgtatgtcc tgacggaaac accgactggc    1200
   agtggtaact catggacgga cacatccttg gtcagctcgt gtctagcaag aaagtag       1257
<210> 12
   <211> 418
   <212> PRT
   <213> Artificial Sequence
<220>
   <223> 108G5
<400> 12
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
   115 120 125
   Asn Asn Tyr Pro Asp Gly Ile Gly Gln Met Gln His Phe Val Asn Asp
   130 135 140
   Asp Gly Met Thr Ile Phe Arg Leu Pro Val Gly Trp Gln Tyr Leu Val
   145 150 155 160
   Asn Asn Asn Leu Gly Gly Asn Leu Asp Ser Thr Ser Ile Ser Lys Tyr
   165 170 175
   Asp Gln Leu Val Gln Gly Cys Leu Ser Leu Gly Ala Tyr Cys Ile Val
   180 185 190
   Asp Ile His Asn Tyr Ala Arg Trp Asn Gly Gly Ile Ile Gly Gln Gly
   195 200 205
   Gly Pro Thr Asn Ala Gln Phe Thr Ser Leu Trp Ser Gln Leu Ala Ser
   210 215 220
   Lys Tyr Ala Ser Gln Ser Arg Val Trp Phe Gly Ile Met Asn Glu Pro
   225 230 235 240
   His Asp Val Asn Ile Asn Thr Trp Ala Ala Thr Val Gln Glu Val Val
   245 250 255
   Thr Ala Ile Arg Asn Ala Gly Ala Thr Ser Gln Phe Ile Ser Leu Pro
   260 265 270
   Gly Asn Asp Trp Gln Ser Ala Gly Ala Phe Ile Ser Asp Gly Ser Ala
   275 280 285
   Ala Ala Leu Ser Gln Val Thr Asn Pro Asp Gly Ser Thr Thr Asn Leu
   290 295 300
   Ile Phe Asp Val His Lys Tyr Leu Asp Ser Asp Asn Ser Gly Thr His
   305 310 315 320
   Ala Glu Cys Thr Thr Asn Asn Ile Asp Gly Ala Phe Ser Pro Leu Ala
   325 330 335
   Thr Trp Leu Arg Ala Asn Gly Arg Gln Ala Ile Leu Thr Glu Thr Gly
   340 345 350
   Gly Gly Asn Val Gln Ser Cys Ile Gln Asp Met Cys Gln Gln Ile Gln
   355 360 365
   Tyr Leu Asn Gln Asn Ser Asp Val Tyr Leu Gly Tyr Val Gly Trp Gly
   370 375 380
   Ala Gly Ser Phe Asp Ser Thr Tyr Val Leu Thr Glu Thr Pro Thr Gly
   385 390 395 400
   Ser Gly Asn Ser Trp Thr Asp Thr Ser Leu Val Ser Ser Cys Leu Ala
   405 410 415
   Arg Lys
<210> 13
   <211> 1257
   <212> DNA
   <213> Artificial Sequence
<220>
   <223> 140F7
<400> 13
   atgaacaagt ccgtggctcc attgctgctt gcagcgtcca tactatatgg cggcgccgct 60
   gcacagcaga ctgtctgggg ccagtgtgga ggtattggtt ggagcggacc tacgaattgt 120
   gctcctggct cagcttgttc gaccctcaat ccttattatg cgcaatgtat tccgggagcc 180
   actactatca ccacttcgac ccggccacca tccggtccaa ccaccaccac cagggctacc 240
   tcaacaagct catcaactcc acccacgagc tctggggtcc gatttgccgg cgttaacatc 300
   gcgggttttg actttggctg taccacagat ggcacttgcg ttacctcgaa ggtttatcct 360
   ccgttgaaga acttcaccgg ctcaaacaac taccccgatg gcatcggcca gatgcagcac 420
   ttcgtcaacg acgacgggat gactattttc cgcttacctg tcggatggca gtacctcgtc 480
   aacaacgttt tgggcggcac acttgattcc aacaatttcg caacctatga ttcacttgtt 540
   caggggtgcc tggcaacagg cgcaagttgc atcattgaca tccacaatta tgctcgatgg 600
   aacggtggga tcattggtca gggcggccct actaatgctc aattcacgag cctttggtcg 660
   cagttggcat caaagtacgc atctcagtcg agggtgtggt tcggcatcat gaatgagccc 720
   cacgacgtga acatcaacac ctgggctgcc acggtccaag aggttgtaac cgcaatccgc 780
   aacgctggtg ctacgtcgca attcatctct ttgcctggaa atgattggca atctgctggg 840
   gctttcatat ccgatggcag tgcagccgcc ctgtctcaag tcacgaaccc ggatgggtca 900
   acaacgaatc tgatttttga cgtgcacaaa tacttggact cagacaactc cggtactcac 960
   gccgaatgta ctacaaataa cattgacggc gccttttctc cgcttgccac ttggctccga 1020
   cagaacaatc gccaggctat cctgacagaa accggtggtg gcaacgttca gtcctgcata 1080
   caagacatgt gccagcaaat ccaatatctc aaccagaact cagatgtcta tcttggctat 1140
   gttggttggg gtgccggatc atttgatagc acgtatgtcc tgacggaaac accgactggc 1200
   agtggtaact catggacgga cacatccttg gtcagctcgt gtctagcaag aaagtag 1257
<210> 14
   <211> 418
   <212> PRT
   <213> Artificial Sequence
<220>
   <223> 140F7
<400> 14
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Val Leu Gly Gly Thr Leu Asp Ser Asn Asn Phe Ala Thr Tyr
                   165                 170                 175
   Asp Ser Leu Val Gln Gly Cys Leu Ala Thr Gly Ala Ser Cys Ile Ile
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305                 310                 315                 320
   Ala Glu Cys Thr Thr Asn Asn Ile Asp Gly Ala Phe Ser Pro Leu Ala
                   325                 330                 335
   Thr Trp Leu Arg Gln Asn Asn Arg Gln Ala Ile Leu Thr Glu Thr Gly
               340                 345                 350
   Gly Gly Asn Val Gln Ser Cys Ile Gln Asp Met Cys Gln Gln Ile Gln
           355                 360                 365
   Tyr Leu Asn Gln Asn Ser Asp Val Tyr Leu Gly Tyr Val Gly Trp Gly
       370                 375                 380
   Ala Gly Ser Phe Asp Ser Thr Tyr Val Leu Thr Glu Thr Pro Thr Gly
   385                 390                 395                 400
   Ser Gly Asn Ser Trp Thr Asp Thr Ser Leu Val Ser Ser Cys Leu Ala
                   405                 410                 415
   Arg Lys
<210> 15
   <211> 1257
   <212> DNA
   <213> Artificial Sequence
<220>
   <223> 146C4
<400> 15
   atgaacaagt ccgtggctcc attgctgctt gcagcgtcca tactatatgg cggcgccgct      60
   gcacagcaga ctgtctgggg ccagtgtgga ggtattggtt ggagcggacc tacgaattgt     120
   gctcctggct cagcttgttc gaccctcaat ccttattatg cgcaatgtat tccgggagcc     180
   actactatca ccacttcgac ccggccacca tccggtccaa ccaccaccac cagggctacc     240
   tcaacaagct catcaactcc acccacgagc tctggggtcc gatttgccgg cgttaacatc     300
   gcgggttttg actttggctg taccacagat ggcacttgcg ttacctcgaa ggtttatcct     360
   ccgttgaaga acttcaccgg ctcaaacaac taccccgatg gcatcggcca gatgcagcac     420
   ttcgtcaacg acgacgggat gactattttc cgcttacctg tcggatggca gtacctcgtc     480
   aacaacaatt tgggcggcaa tcttgattcc acgagcattt ccaagtatga tcagcttgtt     540
   caggggtgcc tgtctctggg cgcatactgc atcgtcgaca tccacaatta tgctcgatgg     600
   aacggtggga tcattggtca gggcggccct actaatgctc aattcacgag cctttggtcg     660
   cagttggcat caaagtacgc atctcagtcg agggtgtggt tcggcatcat gaatgagccc     720
   cacgacgtga acatcaacac ctgggctgcc acggtccaag aggttgtaac cgcaatccgc     780
   aacgctggtg ctacgtcgca attcatctct ttgcctggaa atgattggca atctgctggg     840
   gctttcatat ccgatggcag tgcagccgcc ctgtctaaag tcacgaaccc ggatgggaca     900
   atcacgaatc tgatttttga cgtgcacaaa tacttggact cagacaactc cggtactaat     960
   cttgaatgtg tgacaaataa cattgacggc gccttttctc cgcttgccac ttggctccga    1020
   cagaacaatc gccaggctat cctgacagaa accggtggtg gcaacgttca gtcctgcata    1080
   caagacatgt gccagcaaat ccaatatctc aaccagaact cagatgtcta tcttggctat    1140
   gttggttggg gtgccggatc atttgatagc acgtatgtcc tgacggaaac accgactggc    1200
   agtggtaact catggacgga cacatccttg gtcagctcgt gtctagcaag aaagtag       1257
<210> 16
   <211> 418
   <212> PRT
   <213> Artificial Sequence
<220>
   <223> 146C4
<400> 16
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
                   260                 265                 270
   Gly Asn Asp Trp Gln Ser Ala Gly Ala Phe Ile Ser Asp Gly Ser Ala
           275                 280                 285
   Ala Ala Leu Ser Lys Val Thr Asn Pro Asp Gly Thr Ile Thr Asn Leu
       290                 295                 300
   Ile Phe Asp Val His Lys Tyr Leu Asp Ser Asp Asn Ser Gly Thr Asn
   305                 310                 315                 320
   Leu Glu Cys Val Thr Asn Asn Ile Asp Gly Ala Phe Ser Pro Leu Ala
                   325                 330                 335
   Thr Trp Leu Arg Gln Asn Asn Arg Gln Ala Ile Leu Thr Glu Thr Gly
               340                 345                 350
   Gly Gly Asn Val Gln Ser Cys Ile Gln Asp Met Cys Gln Gln Ile Gln
           355                 360                 365
   Tyr Leu Asn Gln Asn Ser Asp Val Tyr Leu Gly Tyr Val Gly Trp Gly
       370                 375                 380
   Ala Gly Ser Phe Asp Ser Thr Tyr Val Leu Thr Glu Thr Pro Thr Gly
   385                 390                 395                 400
   Ser Gly Asn Ser Trp Thr Asp Thr Ser Leu Val Ser Ser Cys Leu Ala
                   405                 410                 415
   Arg Lys
<210> 17
   <211> 1257
   <212> DNA
   <213> Artificial Sequence
<220>
   <223> 149E4
<400> 17
   atgaacaagt ccgtggctcc attgctgctt gcagcgtcca tactatatgg cggcgccgct      60
   gcacagcaga ctgtctgggg ccagtgtgga ggtattggtt ggagcggacc tacgaattgt     120
   gctcctggct cagcttgttc gaccctcaat ccttattatg cgcaatgtat tccgggagcc     180
   actactatca ccacttcgac ccggccacca tccggtccaa ccaccaccac cagggctacc     240
   tcaacaagct catcaactcc acccacgagc tctggggtcc gatttgccgg cgttaacatc     300
   gcgggttttg actttggctg taccacagat ggcacttgcg ttacctcgaa ggtttatcct     360
   ccgttgaaga acttcaccgg ctcaaacaac taccccgatg gcatcggcca gatgtcccac     420
   ttcgtcaacg acgacgggat gactattttc cgcttacctg tcggatggca gtacctcgtc     480
   aacaacaatt tgggcggcaa tcttgattcc acgagcattt ccaagtatga tcagcttgtt     540
   caggggtgcc tgtctctggg cgcatactgc atcgtcgaca tccacaatta tgctcgatgg     600
   aacggtggga tcattggtca gggcggccct actaatgctc aattcacgag cctttggtcg     660
   cagttggcat caaagtacgc atctcagtcg agggtgtggt tcggcatcat gaatgagccc     720
   cacgacgtga acatcaacac ctgggctgcc acggtccaag aggttgtaac cgcaatccgc     780
   aacgctggtg ctacgtcgca attcatctct ttgcctggaa atgattggca atctgctggg     840
   gctttcatat ccgatggcag tgcagccgcc ctgtctcaag tcacgaaccc ggatgggtca     900
   acaacgaatc tgatttttga cgtgcacaaa tacttggact cagacaactc cggtactcac     960
   gccgaatgtg taacaaataa cattgacgcc gcctttgcac cgcttgccac ttggctccga    1020
   gcaaacggtc gccaggctat cctgagcgaa accggtggtg gcaacaccgc gtcctgccag    1080
   acatatttgt gccagcaagt tgcttatctc aacgcaaact cagatgtcta tcttggctat    1140
   attggttggt ctgccggatc atttgatagc acgtatgtcc tgacggaaac accgactggc    1200
   agtggtaact catggacgga cacatccttg gtcagctcgt gtctagcaag aaagtag       1257
<210> 18
   <211> 418
   <212> PRT
   <213> Artificial Sequence
<220>
   <223> 149E4
<400> 18
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Ser His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305                 310                 315                 320
   Ala Glu Cys Val Thr Asn Asn Ile Asp Ala Ala Phe Ala Pro Leu Ala
                   325                 330                 335
   Thr Trp Leu Arg Ala Asn Gly Arg Gln Ala Ile Leu Ser Glu Thr Gly
   340 345 350
   Gly Gly Asn Thr Ala Ser Cys Gln Thr Tyr Leu Cys Gln Gln Val Ala
   355 360 365
   Tyr Leu Asn Ala Asn Ser Asp Val Tyr Leu Gly Tyr Ile Gly Trp Ser
   370 375 380
   Ala Gly Ser Phe Asp Ser Thr Tyr Val Leu Thr Glu Thr Pro Thr Gly
   385 390 395 400
   Ser Gly Asn Ser Trp Thr Asp Thr Ser Leu Val Ser Ser Cys Leu Ala
   405 410 415
   Arg Lys
<210> 19
   <211> 1257
   <212> DNA
   <213> Artificial Sequence
<220>
   <223> 173C6
<400> 19
   atgaacaagt ccgtggctcc attgctgctt gcagcgtcca tactatatgg cggcgccgct 60
   gcacagcaga ctgtctgggg ccagtgtgga ggtattggtt ggagcggacc tacgaattgt 120
   gctcctggct cagcttgttc gaccctcaat ccttattatg cgcaatgtat tccgggagcc 180
   actactatca ccacttcgac ccggccacca tccggtccaa ccaccaccac cagggctacc 240
   tcaacaagct catcaactcc acccacgagc tctggggtcc gatttgccgg cgttaacatc 300
   gcgggttttg actttggctg taccacagat ggcacttgcg ttacctcgaa ggtttatcct 360
   ccgttgaaga acttcaccgg ctcaaacaac taccccgatg gcatcggcca gatgcagcac 420
   ttcgtcaacg acgacgggat gactattttc cgcttacctg tcggatggca gtacctcgtc 480
   aacaacaatt tgggcggcaa tcttgattcc acgagcattt ccaagtatga tcagcttgtt 540
   caggggtgcc tgtctctggg cgcatactgc atcgtcgaca tccacaatta tgctcgatgg 600
   aacggtggga tcattggtca gggcggccct actaatgctc aattcacgag cctttggtcg 660
   cagttggcat caaagtacgc atctcagtcg agggtgtggt tcggcatcat gaatgagccc 720
   cacgacgtga acatcaacac ctgggctgcc acggtccaag aggttgtaac cgcaatccgc 780
   aacgctggtg ctacgtcgca attcatctct ttgcctggaa atgattggca atctgctggg 840
   gctttcatat ccgatggcag tgcagccgcc ctgtctcaag tcacgaaccc ggatgggtca 900
   acaacgaatc tgatttttga cgtgcacaaa tacttggact cagacaactc cggtactcac 960
   gccgaatgta ctacaaataa cattgacacc gcctttgcac cgcttgccac ttggctccga 1020
   gcaaacggac gccaggctat cctgacagaa accggtggtg gcaacgttgc ttcctgcgag 1080
   acatatttgt gccaggaagt tgcttatctc aacgccaact cagatgtcta tcttggctat 1140
   gttggttggg gtgccggatc atttgatagc acgtatgtcc tgacggaaac accgactggc 1200
   agtggtaact catggacgga cacatccttg gtcagctcgt gtctagcaag aaagtag 1257
<210> 20
   <211> 418
   <212> PRT
   <213> Artificial Sequence
<220>
   <223> 173C6
<400> 20
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305                 310                 315                 320
   Ala Glu Cys Thr Thr Asn Asn Ile Asp Thr Ala Phe Ala Pro Leu Ala
                   325                 330                 335
   Thr Trp Leu Arg Ala Asn Gly Arg Gln Ala Ile Leu Thr Glu Thr Gly
               340                 345                 350
   Gly Gly Asn Val Ala Ser Cys Glu Thr Tyr Leu Cys Gln Glu Val Ala
           355                 360                 365
   Tyr Leu Asn Ala Asn Ser Asp Val Tyr Leu Gly Tyr Val Gly Trp Gly
       370                 375                 380
   Ala Gly Ser Phe Asp Ser Thr Tyr Val Leu Thr Glu Thr Pro Thr Gly
   385                 390                 395                 400
   Ser Gly Asn Ser Trp Thr Asp Thr Ser Leu Val Ser Ser Cys Leu Ala
                   405                 410                 415
   Arg Lys
<210> 21
   <211> 1257
   <212> DNA
   <213> Artificial Sequence
<220>
   <223> 191H11
<400> 21
   atgaacaagt ccgtggctcc attgctgctt gcagcgtcca tactatatgg cggcgccgct      60
   gcacagcaga ctgtctgggg ccagtgtgga ggtattggtt ggagcggacc tacgaattgt     120
   gctcctggct cagcttgttc gaccctcaat ccttattatg cgcaatgtat tccgggagcc     180
   actactatca ccacttcgac ccggccacca tccggtccaa ccaccaccac cagggctacc     240
   tcaacaagct catcaactcc acccacgagc tctggggtcc gatttgccgg cgttaacatc     300
   gcgggttttg actttggctg taccacagat ggcacttgcg ttacctcgaa ggtttatcct     360
   ccgttgaaga acttcaccgg ctcaaacaac taccccgatg gcatcggcca gatgcagcac     420
   ttcgtcaacg acgacgggat gactattttc cgcttacctg tcggatggca gtacctcgtc     480
   aacaacaatt tgggcggcaa tcttgattcc acgagcattt ccaagtatga tcagcttgtt     540
   caggggtgcc tgtctctggg cgcatactgc atcgtcgaca tccacaatta tgctcgatgg     600
   aacggtggga tcattggtca gggcggccct actaatgctc aattcacgag cctttggtcg     660
   cagttggcat caaagtacgc atctcagtcg agggtgtggt tcggcatcat gaatgagccc     720
   cacgacgtga acatcaacac ctgggctgcc acggtccaag aggttgtaac cgcaatccgc     780
   aacgctggtg ctacgtcgca attcatctct ttgcctggaa atgattggca atctgctggg     840
   gctttcatat ccgatggcag tgcagccgcc ctgtctcaag tcacgaaccc ggatgggtca     900
   acaacgaatc tgatttttga cgtgcacaaa tacttggact cagacaactc cggtactcac     960
   gccgaatgta ctacaaataa cattgacgcc gcctttgcac cgcttgccac ttggctccga    1020
   gcaaacggtc gccaggctat cctgagcgaa accggtggtg gcaacaccgc gtcctgccag    1080
   acatatttgt gccagcaagt tgcttatctc aacgcaaact cagatgtcta tcttggctat    1140
   attggttggt ctgccggatc atttgatagc acgtatgtcc tgacggaaac accgactggc    1200
   agtggtaact catggacgga cacatccttg gtcagctcgt gtctagcaag aaagtag       1257
<210> 22
   <211> 418
   <212> PRT
   <213> Artificial Sequence
<220>
   <223> 191H11
<400> 22
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305                 310                 315                 320
   Ala Glu Cys Thr Thr Asn Asn Ile Asp Ala Ala Phe Ala Pro Leu Ala
                   325                 330                 335
   Thr Trp Leu Arg Ala Asn Gly Arg Gln Ala Ile Leu Ser Glu Thr Gly
               340                 345                 350
   Gly Gly Asn Thr Ala Ser Cys Gln Thr Tyr Leu Cys Gln Gln Val Ala
           355                 360                 365
   Tyr Leu Asn Ala Asn Ser Asp Val Tyr Leu Gly Tyr Ile Gly Trp Ser
       370                 375                 380
   Ala Gly Ser Phe Asp Ser Thr Tyr Val Leu Thr Glu Thr Pro Thr Gly
   385                 390                 395                 400
   Ser Gly Asn Ser Trp Thr Asp Thr Ser Leu Val Ser Ser Cys Leu Ala
                   405                 410                 415
   Arg Lys
<210> 23
   <211> 1260
   <212> DNA
   <213> Artificial Sequence
<220>
   <223> 222E1
<400> 23
   atgaacaagt ccgtggctcc attgctgctt gcagcgtcca tactatatgg cggcgccgct 60
   gcacagcaga ctgtctgggg ccagtgtgga ggtattggtt ggagcggacc tacgaattgt 120
   gctcctggct cagcttgttc gaccctcaat ccttattatg cgcaatgtat tccgggagcc 180
   actactatca ccacttcgac ccggccacca tccggtccaa ccaccaccac cagggctacc 240
   tcaacaagct catcaactcc acccacgagc tctggggtcc gatttgccgg cgttaacatc 300
   gcgggttttg actttggctg taccacagat ggcacttgcg ttacctcgaa ggtttatcct 360
   ccgttgaaga acttcaccgg ctcaaacaac taccccgatg gcatcggcca gatgcagcac 420
   ttcgtcaacg acgacgggat gactattttc cgcttacctg tcggatggca gtacctcgtc 480
   aacaacaatt tgggcggcaa tcttgattcc acgagcattt ccaagtatga tcagcttgtt 540
   caggggtgcc tgtctctggg cgcatactgc atcgtcgaca tccacaatta tgctcgatgg 600
   aacggtggga tcattggtca gggcggccct actaatgctc aattcacgag cctttggtcg 660
   cagttggcat caaagtacgc atctcagtcg agggtgtggt tcggcatcat gaatgagccc 720
   cacgacgtga acatcaacac ctgggctgcc acggtccaag aggttgtaac cgcaatccgc 780
   aacgctggtg ctacgtcgca attcatctct ttgcctggaa atgattggca atctgctggg 840
   gctttcatat ccgatggcag tgcagccgcc ctgtctcaag tcacgaaccc ggatgggtca 900
   acaacgaatc tgatttttga cgtgcacaaa tacttggact cagacaactc cggtactcac 960
   gccgaatgtg taacaaataa cattgacgcc gcctttgcac cgcttgccac ttggctccga 1020
   gcaaacggac gccaggctat cctgacagaa accggtggtg gcaacgttgc ttcctgcgag 1080
   acatatttgt gccaggaagt tgcttatctc aacgcaaact cagatgtcta tcttggctat 1140
   gttggttggg ctgccggatc atttgatacg aattatacac tgacggaaac accgaatggc 1200
   agtggttcat caatgacgga ccaaccattg gtcgcggctt gtctaactag atcgaattag 1260
<210> 24
   <211> 419
   <212> PRT
   <213> Artificial Sequence
<220>
   <223> 222E1
<400> 24
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65 70 75 80
   Ser Thr Ser Ser Ser Thr Pro Pro Thr Ser Ser Gly Val Arg Phe Ala
   85 90 95
   Gly Val Asn Ile Ala Gly Phe Asp Phe Gly Cys Thr Thr Asp Gly Thr
   100 105 110
   Cys Val Thr Ser Lys Val Tyr Pro Pro Leu Lys Asn Phe Thr Gly Ser
   115 120 125
   Asn Asn Tyr Pro Asp Gly Ile Gly Gln Met Gln His Phe Val Asn Asp
   130 135 140
   Asp Gly Met Thr Ile Phe Arg Leu Pro Val Gly Trp Gln Tyr Leu Val
   145 150 155 160
   Asn Asn Asn Leu Gly Gly Asn Leu Asp Ser Thr Ser Ile Ser Lys Tyr
   165 170 175
   Asp Gln Leu Val Gln Gly Cys Leu Ser Leu Gly Ala Tyr Cys Ile Val
   180 185 190
   Asp Ile His Asn Tyr Ala Arg Trp Asn Gly Gly Ile Ile Gly Gln Gly
   195 200 205
   Gly Pro Thr Asn Ala Gln Phe Thr Ser Leu Trp Ser Gln Leu Ala Ser
   210 215 220
   Lys Tyr Ala Ser Gln Ser Arg Val Trp Phe Gly Ile Met Asn Glu Pro
   225 230 235 240
   His Asp Val Asn Ile Asn Thr Trp Ala Ala Thr Val Gln Glu Val Val
   245 250 255
   Thr Ala Ile Arg Asn Ala Gly Ala Thr Ser Gln Phe Ile Ser Leu Pro
   260 265 270
   Gly Asn Asp Trp Gln Ser Ala Gly Ala Phe Ile Ser Asp Gly Ser Ala
   275 280 285
   Ala Ala Leu Ser Gln Val Thr Asn Pro Asp Gly Ser Thr Thr Asn Leu
   290 295 300
   Ile Phe Asp Val His Lys Tyr Leu Asp Ser Asp Asn Ser Gly Thr His
   305 310 315 320
   Ala Glu Cys Val Thr Asn Asn Ile Asp Ala Ala Phe Ala Pro Leu Ala
   325 330 335
   Thr Trp Leu Arg Ala Asn Gly Arg Gln Ala Ile Leu Thr Glu Thr Gly
   340 345 350
   Gly Gly Asn Val Ala Ser Cys Glu Thr Tyr Leu Cys Gln Glu Val Ala
   355 360 365
   Tyr Leu Asn Ala Asn Ser Asp Val Tyr Leu Gly Tyr Val Gly Trp Ala
   370 375 380
   Ala Gly Ser Phe Asp Thr Asn Tyr Thr Leu Thr Glu Thr Pro Asn Gly
   385 390 395 400
   Ser Gly Ser Ser Met Thr Asp Gln Pro Leu Val Ala Ala Cys Leu Thr
   405 410 415
   Arg Ser Asn
<210> 25
   <211> 1260
   <212> DNA
   <213> Artificial Sequence
<220>
   <223> 225C7
<400> 25
   atgaacaagt ccgtggctcc attgctgctt gcagcgtcca tactatatgg cggcgccgct 60
   gcacagcaga ctgtctgggg ccagtgtgga ggtattggtt ggagcggacc tacgaattgt 120
   gctcctggct cagcttgttc gaccctcaat ccttattatg cgcaatgtat tccgggagcc 180
   actactatca ccacttcgac ccggccacca tccggtccaa ccaccaccac cagggctacc 240
   tcaacaagct catcaactcc acccacgagc tctggggtcc gatttgccgg cgttaacatc 300
   gcgggttttg actttggctg taccacagat ggcacttgcg ttacctcgaa ggtttatcct 360
   ccgttgaaga acttcaccgg ctcaaacaac taccccgatg gcctcggcca gatgtcccac 420
   ttcgtcaaaa gcacagggca caatattttc cgcttacctg tcggatggca gtacctcgtc 480
   aacaacgttt tgggcggcac acttgattcc aacaatttcg caacctatga ttcacttgtt 540
   caggggtgcc tggcaacagg cgcaagttgc atcattgaca tccacaatta tgctcgatgg 600
   aacggtggga tcattggtca gggcggccct actaatgctc aattcacgag cctttggtcg 660
   cagttggcat caaagtacgc atctcagtcg agggtgtggt tcggcatcat gaatgagccc 720
   cacgacgtga acatcaacac ctgggctgcc acggtccaag aggttgtaac cgcaatccgc      780
   aacgctggtg ctacgtcgca attcatctct ttgcctggaa atgattggca atctgctggg      840
   gctttcatat ccgatggcag tgcagccgcc ctgtctcaag tcacgaaccc ggatgggtca      900
   acaacgaatc tgatttttga cgtgcacaaa tacttggact cagacaactc cggtactcac      960
   gccgaatgta ctacaaataa cattgacggc gccttttctc cgcttgccac ttggctccga     1020
   gcaaacggac gccaggctat cctgagcgaa accggtggtg gcaacaccgc gtcctgccag     1080
   acatatttgt gccagcaagt tgcttatctc aacgcaaact cagatgtcta tcttggctat     1140
   attggttggt ctgccggatc atttgatagc acgtatattc tgacggaaac accgaatggc     1200
   agtggttctt caatgacgga ccaagcgttg gtcgcggctt gtctaactag aacatcgtag     1260
<210> 26
   <211> 419
   <212> PRT
   <213> Artificial Sequence
<220>
   <223> 225C7
<400> 26
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Leu Gly Gln Met Ser His Phe Val Lys Ser
       130                 135                 140
   Thr Gly His Asn Ile Phe Arg Leu Pro Val Gly Trp Gln Tyr Leu Val
   145                 150                 155                 160
   Asn Asn Val Leu Gly Gly Thr Leu Asp Ser Asn Asn Phe Ala Thr Tyr
                   165                 170                 175
   Asp Ser Leu Val Gln Gly Cys Leu Ala Thr Gly Ala Ser Cys Ile Ile
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305                 310                 315                 320
   Ala Glu Cys Thr Thr Asn Asn Ile Asp Gly Ala Phe Ser Pro Leu Ala
                   325                 330                 335
   Thr Trp Leu Arg Ala Asn Gly Arg Gln Ala Ile Leu Ser Glu Thr Gly
               340                 345                 350
   Gly Gly Asn Thr Ala Ser Cys Gln Thr Tyr Leu Cys Gln Gln Val Ala
           355                 360                 365
   Tyr Leu Asn Ala Asn Ser Asp Val Tyr Leu Gly Tyr Ile Gly Trp Ser
       370                 375                 380
   Ala Gly Ser Phe Asp Ser Thr Tyr Ile Leu Thr Glu Thr Pro Asn Gly
   385 390 395 400
   Ser Gly Ser Ser Met Thr Asp Gln Ala Leu Val Ala Ala Cys Leu Thr
   405 410 415
   Arg Thr Ser
<210> 27
   <211> 1248
   <212> DNA
   <213> Artificial Sequence
<220>
   <223> 227C4
<400> 27
   atgaacaagt ccgtggctcc attgctgctt gcagcgtcca tactatatgg cggcgccgct 60
   gcacagcaga ctgtctgggg ccagtgtgga ggtattggtt ggagcggacc tacgaattgt 120
   gctcctggct cagcttgttc gaccctcaat ccttattatg cgcaatgtat tccgggagcc 180
   actactatca ccacttcgac ccggccacca tccggtccaa ccaccaccac cagggctacc 240
   tcaacaagct catcaactcc acccacgagc tctggggtcc gatttgccgg cgttaacatc 300
   gcgggttttg actttggctg taccacagat ggcacttgcc taaccaataa ggtttatcct 360
   gctttgagtt ccctcaacaa cggccccgat ggcctgggcc agatggccca cttcgtctcc 420
   aaaactgggc ataatatttt ccgcttacct gtcggatggc agtacctcgt caacaacaat 480
   ttgggcggca cacttgattc ctccaacctt gcgacctatg atttgcttgt tcaggggtgc 540
   ctgtctctgg gcgcatactg catcgtcgac atccacaatt atgctcgatg gaacggtggg 600
   atcattggtc agggcggccc tactaatgct caattcacga gcctttggtc gcagttggca 660
   tcaaagtacg catctcagtc gagggtgtgg ttcggcatca tgaatgagcc ccacgacgtg 720
   aacatcaaca cctgggctgc cacggtccaa gaggttgtaa ccgcaatccg caacgctggt 780
   gctacgtcgc aattcatctc tttgcctgga aatgattggc aatctgctgg ggctttcata 840
   tccgatggca gtgcagccgc cctgtctcaa gtcacgaacc cggatgggtc aacaacgaat 900
   ctgatttttg acgtgcacaa atacttggac tcagacaact ccggtactca cgccgaatgt 960
   gtaacaaata acattgacgc cgcctttgca ccgcttgcca cttggctccg agcaaacggt 1020
   cgccaggcta tcctgacaga aaccggtggt ggcaacgttc agtcctgcat acaagacatg 1080
   tgccagcaaa tccaatatct caaccagaac tcagatgtct atcttggcta tgttggttgg 1140
   ggtgccggat catttgatag cacgtatgtc ctgacggaaa caccgactgg cagtggtaac 1200
   tcatggacgg acacatcctt ggtcagctcg tgtctagcaa gaaagtag 1248
<210> 28
   <211> 415
   <212> PRT
   <213> Artificial Sequence
<220>
   <223> 227C4
<400> 28
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Leu Thr Asn Lys Val Tyr Pro Ala Leu Ser Ser Leu Asn Asn Gly
           115                 120                 125
   Pro Asp Gly Leu Gly Gln Met Ala His Phe Val Ser Lys Thr Gly His
       130                 135                 140
   Asn Ile Phe Arg Leu Pro Val Gly Trp Gln Tyr Leu Val Asn Asn Asn
   145                 150                 155                 160
   Leu Gly Gly Thr Leu Asp Ser Ser Asn Leu Ala Thr Tyr Asp Leu Leu
                   165                 170                 175
   Val Gln Gly Cys Leu Ser Leu Gly Ala Tyr Cys Ile Val Asp Ile His
               180                 185                 190
   Asn Tyr Ala Arg Trp Asn Gly Gly Ile Ile Gly Gln Gly Gly Pro Thr
           195                 200                 205
   Asn Ala Gln Phe Thr Ser Leu Trp Ser Gln Leu Ala Ser Lys Tyr Ala
           210                 215                 220
   Ser Gln Ser Arg Val Trp Phe Gly Ile Met Asn Glu Pro His Asp Val
   225                 230                 235                 240
   Asn Ile Asn Thr Trp Ala Ala Thr Val Gln Glu Val Val Thr Ala Ile
                   245                 250                 255
   Arg Asn Ala Gly Ala Thr Ser Gln Phe Ile Ser Leu Pro Gly Asn Asp
               260                 265                 270
   Trp Gln Ser Ala Gly Ala Phe Ile Ser Asp Gly Ser Ala Ala Ala Leu
           275                 280                 285
   Ser Gln Val Thr Asn Pro Asp Gly Ser Thr Thr Asn Leu Ile Phe Asp
       290                 295                 300
   Val His Lys Tyr Leu Asp Ser Asp Asn Ser Gly Thr His Ala Glu Cys
   305                 310                 315                 320
   Val Thr Asn Asn Ile Asp Ala Ala Phe Ala Pro Leu Ala Thr Trp Leu
                   325                 330                 335
   Arg Ala Asn Gly Arg Gln Ala Ile Leu Thr Glu Thr Gly Gly Gly Asn
               340                 345                 350
   Val Gln Ser Cys Ile Gln Asp Met Cys Gln Gln Ile Gln Tyr Leu Asn
           355                 360                 365
   Gln Asn Ser Asp Val Tyr Leu Gly Tyr Val Gly Trp Gly Ala Gly Ser
       370                 375                 380
   Phe Asp Ser Thr Tyr Val Leu Thr Glu Thr Pro Thr Gly Ser Gly Asn
   385                 390                 395                 400
   Ser Trp Thr Asp Thr Ser Leu Val Ser Ser Cys Leu Ala Arg Lys
                   405                 410                 415
<210> 29
   <211> 1257
   <212> DNA
   <213> Artificial Sequence
<220>
   <223> 229D1
<400> 29
   atgaacaagt ccgtggctcc attgctgctt gcagcgtcca tactatatgg cggcgccgct      60
   gcacagcaga ctgtctgggg ccagtgtgga ggtattggtt ggagcggacc tacgaattgt     120
   gctcctggct cagcttgttc gaccctcaat ccttattatg cgcaatgtat tccgggagcc     180
   actactatca ccacttcgac ccggccacca tccggtccaa ccaccaccac cagggctacc     240
   tcaacaagct catcaactcc acccacgagc tctggggtcc gatttgccgg cgttaacatc     300
   gcgggttttg actttggctg taccacagat ggcacttgcg ttacctcgaa ggtttatcct     360
   ccgttgaaga acttcaccgg ctcaaacaac taccccgatg gcatcggcca gatgcagcac     420
   ttcgtcaacg acgacgggat gactattttc cgcttacctg tcggatggca gtacctcgtc     480
   aacaacaatt tgggcggcaa tcttgattcc acgagcattt ccaagtatga tcagcttgtt     540
   caggggtgcc tgtctctggg cgcatactgc atcgtcgaca tccacaatta tgctcgatgg     600
   aacggtggga tcattggtca gggcggccct actaatgctc aattcacgag cctttggtcg     660
   cagttggcat caaagtacgc atctcagtcg agggtgtggt tcggcatcat gaatgagccc     720
   cacgacgtga acatcaacac ctgggctgcc acggtccaag aggttgtaac cgcaatccgc     780
   aacgctggtg ctacgtcgca attcatctct ttgcctggaa atgattggca atctgctggg     840
   gctttcatat ccgatggcag tgcagccgcc ctgtctcaag tcacgaaccc ggatgggtca     900
   acaacgaatc tgatttttga cgtgcacaaa tacttggact cagacaactc cggtactcac     960
   gccgaatgta ctacaaataa cattgacggc gccttttctc cgcttgccac ttggctccga    1020
   cagaacaatc gccaggctat cctgacagaa accggtggtg gcaacaccgc gtcctgccag    1080
   acatatttgt gccagcaagt tgcttatctc aacgcaaact cagatgtcta tcttggctat    1140
   attggttggt ctgccggatc atttgatagc acgtatattc tgacggaaac accgactggc    1200
   agtggtaact catggacgga cacatccttg gtcagctcgt gtctagcaag aaagtag       1257
<210> 30
   <211> 418
   <212> PRT
   <213> Artificial Sequence
<220>
   <223> 229D1
<400> 30
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305 310 315 320
   Ala Glu Cys Thr Thr Asn Asn Ile Asp Gly Ala Phe Ser Pro Leu Ala
   325 330 335
   Thr Trp Leu Arg Gln Asn Asn Arg Gln Ala Ile Leu Thr Glu Thr Gly
   340 345 350
   Gly Gly Asn Thr Ala Ser Cys Gln Thr Tyr Leu Cys Gln Gln Val Ala
   355 360 365
   Tyr Leu Asn Ala Asn Ser Asp Val Tyr Leu Gly Tyr Ile Gly Trp Ser
   370 375 380
   Ala Gly Ser Phe Asp Ser Thr Tyr Ile Leu Thr Glu Thr Pro Thr Gly
   385 390 395 400
   Ser Gly Asn Ser Trp Thr Asp Thr Ser Leu Val Ser Ser Cys Leu Ala
   405 410 415
   Arg Lys
<210> 31
   <211> 1257
   <212> DNA
   <213> Artificial Sequence
<220>
   <223> 231C9
<400> 31
   atgaacaagt ccgtggctcc attgctgctt gcagcgtcca tactatatgg cggcgccgct       60
   gcacagcaga ctgtctgggg ccagtgtgga ggtattggtt ggagcggacc tacgaattgt      120
   gctcctggct cagcttgttc gaccctcaat ccttattatg cgcaatgtat tccgggagcc      180
   actactatca ccacttcgac ccggccacca tccggtccaa ccaccaccac cagggctacc      240
   tcaacaagct catcaactcc acccacgagc tctggggtcc gatttgccgg cgttaacatc      300
   gcgggttttg actttggctg taccacagat ggcacttgcg ttacctcgaa ggtttatcct      360
   ccgttgaaga acttcaccgg ctcaaacaac taccccgatg gcatcggcca gatgcagcac      420
   ttcgtcaacg acgacgggat gactattttc cgcttacctg tcggatggca gtacctcgtc      480
   aacaacaatt tgggcggcaa tcttgattcc acgagcattt ccaagtatga tcagcttgtt      540
   caggggtgcc tgtctctggg cgcatactgc atcgtcgaca tccacaatta tgctcgatgg      600
   aacggtggga tcattggtca gggcggccct actaatgctc aattcacgag cctttggtcg      660
   cagttggcat caaagtacgc atctcagtcg agggtgtggt tcggcatcat gaatgagccc      720
   cacgacgtga acatcaacac ctgggctgcc acggtccaag aggttgtaac cgcaatccgc      780
   aacgctggtg ctacgtcgca attcatctct ttgcctggaa atgattggca atctgctggg      840
   gctttcatat ccgatggcag tgcagccgcc ctgtctcaag tcacgaaccc ggatgggtca      900
   acaacgaatc tgatttttga cgtgcacaaa tacttggact cagacaactc cggtactcac      960
   gccgaatgtg taacaaataa cattgacgcc gcctttgcac cgcttgccac ttggctccga     1020
   gcaaacggtc gccaggctat cctgagcgaa accggtggtg gcaacaccgc gtcctgccag     1080
   acatatttgt gccagcaagt tgcttatctc aacgcaaact cagatgtcta tcttggctat     1140
   attggttggg gtgccggatc atttgatagc acgtatgtcc tgacggaaac accgactggc     1200
   agtggtaact catggacgga cacatccttg gtcagctcgt gtctagcaag aaagtag        1257
<210> 32
   <211> 418
   <212> PRT
   <213> Artificial Sequence
<220>
   <223> 231C9
<400> 32
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305                 310                 315                 320
   Ala Glu Cys Val Thr Asn Asn Ile Asp Ala Ala Phe Ala Pro Leu Ala
                   325                 330                 335
   Thr Trp Leu Arg Ala Asn Gly Arg Gln Ala Ile Leu Ser Glu Thr Gly
               340                 345                 350
   Gly Gly Asn Thr Ala Ser Cys Gln Thr Tyr Leu Cys Gln Gln Val Ala
           355                 360                 365
   Tyr Leu Asn Ala Asn Ser Asp Val Tyr Leu Gly Tyr Ile Gly Trp Gly
       370                 375                 380
   Ala Gly Ser Phe Asp Ser Thr Tyr Val Leu Thr Glu Thr Pro Thr Gly
   385                 390                 395                 400
   Ser Gly Asn Ser Trp Thr Asp Thr Ser Leu Val Ser Ser Cys Leu Ala
                   405                 410                 415
   Arg Lys
<210> 33
   <211> 1260
   <212> DNA
   <213> Artificial Sequence
<220>
   <223> 330F9
<400> 33
   atgaacaagt ccgtggctcc attgctgctt gcagcgtcca tactatatgg cggcgccgct      60
   gcacagcaga ctgtctgggg ccagtgtgga ggtattggtt ggagcggacc tacgaattgt     120
   gctcctggct cagcttgttc gaccctcaat ccttattatg cgcaatgtat tccgggagcc     180
   actactatca ccacttcgac ccggccacca tccggtccaa ccaccaccac cagggctacc     240
   tcaacaagct catcaactcc acccacgagc tctggggtcc gatttgccgg cgttaacatc     300
   gcgggttttg actttggctg taccacagat ggcacttgcg ttacctcgaa ggtttatcct     360
   ccgttgaaga acttcaccgg ctcaaacaac taccccgatg gcatcggcca gatgcagcac     420
   ttcgtcaacg acgacgggat gactattttc cgcttacctg tcggatggca gtacctcgtc     480
   aacaacaatt tgggcggcaa tcttgattcc acgagcattt ccaagtatga tcagcttgtt     540
   caggggtgcc tgtctctggg cgcatactgc atcgtcgaca tccacaatta tgctcgatgg     600
   aacggtggga tcattggtca gggcggccct actaatgctc aattcacgag cctttggtcg     660
   cagttggcat caaagtacgc atctcagtcg agggtgtggt tcggcatcat gaatgagccc     720
   cacgacgtga acatcaacac ctgggctgcc acggtccaag aggttgtaac cgcaatccgc     780
   aacgctggtg ctacgtcgca attcatctct ttgcctggaa atgattggca atctgctggg     840
   gctttcatat ccgatggcag tgcagccgcc ctgtctcaag tcacgaaccc ggatgggtca     900
   acaacgaatc tgatttttga cgtgcacaaa tacttggact cagacaactc cggtactcac     960
   gccgaatgtg taacaaataa cattgacgcc gcctttgcac cgcttgccac ttggctccga    1020
   gcaaacggtc gccaggctat cctgagcgaa accggtggtg gcaacaccgc gtcctgccag    1080
   acatatttgt gccagcaagt tgcttatctc aacgcaaact cagatgtcta tcttggctat    1140
   gttggttggg ctgccggatc atttgatacg aattatacac tgacggaaac accgaatggc    1200
   agtggttctt caatgacgga ccaagcgttg gtcgcggctt gtctaactag atcgaattag    1260
<210> 34
   <211> 419
   <212> PRT
   <213> Artificial Sequence
<220>
   <223> 330F9
<400> 34
   Met Asn Lys Ser Val Ala Pro Leu Leu Leu Ala Ala Ser Ile Leu Tyr
   1               5                   10                  15
   Gly Gly Ala Ala Ala Gln Gln Thr Val Trp Gly Gln Cys Gly Gly Ile
               20                  25                  30
   Gly Trp Ser Gly Pro Thr Asn Cys Ala Pro Gly Ser Ala Cys Ser Thr
           35                  40                  45
   Leu Asn Pro Tyr Tyr Ala Gln Cys Ile Pro Gly Ala Thr Thr Ile Thr
       50                  55                  60
   Thr Ser Thr Arg Pro Pro Ser Gly Pro Thr Thr Thr Thr Arg Ala Thr
   65                  70                  75                  80
   Ser Thr Ser Ser Ser Thr Pro Pro Thr Ser Ser Gly Val Arg Phe Ala
                   85                  90                  95
   Gly Val Asn Ile Ala Gly Phe Asp Phe Gly Cys Thr Thr Asp Gly Thr
               100                 105                 110
   Cys Val Thr Ser Lys Val Tyr Pro Pro Leu Lys Asn Phe Thr Gly Ser
           115                 120                 125
   Asn Asn Tyr Pro Asp Gly Ile Gly Gln Met Gln His Phe Val Asn Asp
       130                 135                 140
   Asp Gly Met Thr Ile Phe Arg Leu Pro Val Gly Trp Gln Tyr Leu Val
   145                 150                 155                 160
   Asn Asn Asn Leu Gly Gly Asn Leu Asp Ser Thr Ser Ile Ser Lys Tyr
                   165                 170                 175
   Asp Gln Leu Val Gln Gly Cys Leu Ser Leu Gly Ala Tyr Cys Ile Val
               180                 185                 190
   Asp Ile His Asn Tyr Ala Arg Trp Asn Gly Gly Ile Ile Gly Gln Gly
           195                 200                 205
   Gly Pro Thr Asn Ala Gln Phe Thr Ser Leu Trp Ser Gln Leu Ala Ser
       210                 215                 220
   Lys Tyr Ala Ser Gln Ser Arg Val Trp Phe Gly Ile Met Asn Glu Pro
   225                 230                 235                 240
   His Asp Val Asn Ile Asn Thr Trp Ala Ala Thr Val Gln Glu Val Val
                   245                 250                 255
   Thr Ala Ile Arg Asn Ala Gly Ala Thr Ser Gln Phe Ile Ser Leu Pro
               260                 265                 270
   Gly Asn Asp Trp Gln Ser Ala Gly Ala Phe Ile Ser Asp Gly Ser Ala
           275                 280                 285
   Ala Ala Leu Ser Gln Val Thr Asn Pro Asp Gly Ser Thr Thr Asn Leu
       290                 295                 300
   Ile Phe Asp Val His Lys Tyr Leu Asp Ser Asp Asn Ser Gly Thr His
   305                 310                 315                 320
   Ala Glu Cys Val Thr Asn Asn Ile Asp Ala Ala Phe Ala Pro Leu Ala
                   325                 330                 335
   Thr Trp Leu Arg Ala Asn Gly Arg Gln Ala Ile Leu Ser Glu Thr Gly
               340                 345                 350
   Gly Gly Asn Thr Ala Ser Cys Gln Thr Tyr Leu Cys Gln Gln Val Ala
           355                 360                 365
   Tyr Leu Asn Ala Asn Ser Asp Val Tyr Leu Gly Tyr Val Gly Trp Ala
       370                 375                 380
   Ala Gly Ser Phe Asp Thr Asn Tyr Thr Leu Thr Glu Thr Pro Asn Gly
   385                 390                 395                 400
   Ser Gly Ser Ser Met Thr Asp Gln Ala Leu Val Ala Ala Cys Leu Thr
                   405                 410                 415
   Arg Ser Asn
<210> 35
   <211> 1236
   <212> DNA
   <213> Botryotinia fuckeliana
<400> 35
   atgaagctct ccaccaccgt atacacagtt gttcctttcc tgtctaccgc caccgcacag      60
   ggcgttgctt acgcacagtg tggaggtaat ggttggactg gatctacggc ttgtgtgtca     120
   ggctatgctt gttcgtatgt gaatgcgtat tattcgcaat gtttgccggg aactgcaact     180
   ttaaccactg ttaccagtgc aaccaccagt gctagttcga aaacaagcac agccgcagca     240
   cccaatagca cggggaagac aaaatatatc ggcaccaaca tcgcgggttt tgactttggc     300
   tgtaccacag atggcacttg cctaaccaat aaggtttatc ctgctttgag ttccctcaac     360
   aacggccccg atggcctggg ccagatggcc cacttcgtct ccaaaactgg gcataatatt     420
   ttccgcttac ctgtcggatg gcagtacctc gtcaacaaca atttgggcgg cacacttgat     480
   tcctccaacc ttgcgaccta tgatttgctt gttcaggggt gcctggcaac gggcgcaact     540
   tgcgtgattg acatccacaa ttatgctcga tggaacggtg caatcattgg tcagggcggc     600
   cctactgatg ctcaattcgc tagcctttgg tcgcagttgg caacgaagta caagtctaat     660
   acgaaggtgg tcttcggctt gatgaatgag ccccacgact tgaacagcat caccacctgg     720
   gctgccacgc ttcaaacagt tgtaaccgca atccgccagg ctggtgctac gtcgaccatg     780
   cttctattgc ctggaagtga ttacacatct gctggggctt tcataaccga tggcagtgca     840
   gccgccctgt ctaagatcac gaacctcgat gggactacaa cgaatctgat ttttgacgtg      900
   cacaaatact tggactcaga caactccggt actcacgccg aatgtgtaac aaataacatt     960
   gacgccgcct ttgcaccgct tgccacttgg ctccgagcaa acggtcgcca ggctatcctg    1020
   agcgaaaccg gtggtggcaa caccgcgtcc tgccagacat atttgtgcca gcaagttgct    1080
   tatctcaacg caaactcaga tgtctatctt ggctatattg gttggtctgc cggatcattt    1140
   gatagcacgt atattctgac ggaaacaccg aatggcagtg gttcttcaat gacggaccaa    1200
   gcgttggtcg cggcttgtct aactagaaca tcgtag                              1236
<210> 36
   <211> 411
   <212> PRT
   <213> Botryotinia fuckeliana
<400> 36
   Met Lys Leu Ser Thr Thr Val Tyr Thr Val Val Pro Phe Leu Ser Thr
   1               5                   10                  15
   Ala Thr Ala Gln Gly Val Ala Tyr Ala Gln Cys Gly Gly Asn Gly Trp
               20                  25                  30
   Thr Gly Ser Thr Ala Cys Val Ser Gly Tyr Ala Cys Ser Tyr Val Asn
           35                  40                  45
   Ala Tyr Tyr Ser Gln Cys Leu Pro Gly Thr Ala Thr Leu Thr Thr Val
       50                  55                  60
   Thr Ser Ala Thr Thr Ser Ala Ser Ser Lys Thr Ser Thr Ala Ala Ala
   65                  70                  75                  80
   Pro Asn Ser Thr Gly Lys Thr Lys Tyr Ile Gly Thr Asn Ile Ala Gly
                   85                  90                  95
   Phe Asp Phe Gly Cys Thr Thr Asp Gly Thr Cys Leu Thr Asn Lys Val
               100                 105                 110
   Tyr Pro Ala Leu Ser Ser Leu Asn Asn Gly Pro Asp Gly Leu Gly Gln
           115                 120                 125
   Met Ala His Phe Val Ser Lys Thr Gly His Asn Ile Phe Arg Leu Pro
       130                 135                 140
   Val Gly Trp Gln Tyr Leu Val Asn Asn Asn Leu Gly Gly Thr Leu Asp
   145                 150                 155                 160
   Ser Ser Asn Leu Ala Thr Tyr Asp Leu Leu Val Gln Gly Cys Leu Ala
                   165                 170                 175
   Thr Gly Ala Thr Cys Val Ile Asp Ile His Asn Tyr Ala Arg Trp Asn
               180                 185                 190
   Gly Ala Ile Ile Gly Gln Gly Gly Pro Thr Asp Ala Gln Phe Ala Ser
           195                 200                 205
   Leu Trp Ser Gln Leu Ala Thr Lys Tyr Lys Ser Asn Thr Lys Val Val
       210                 215                 220
   Phe Gly Leu Met Asn Glu Pro His Asp Leu Asn Ser Ile Thr Thr Trp
   225                 230                 235                 240
   Ala Ala Thr Leu Gln Thr Val Val Thr Ala Ile Arg Gln Ala Gly Ala
                   245                 250                 255
   Thr Ser Thr Met Leu Leu Leu Pro Gly Ser Asp Tyr Thr Ser Ala Gly
               260                 265                 270
   Ala Phe Ile Thr Asp Gly Ser Ala Ala Ala Leu Ser Lys Ile Thr Asn
           275                 280                 285
   Leu Asp Gly Thr Thr Thr Asn Leu Ile Phe Asp Val His Lys Tyr Leu
       290                 295                 300
   Asp Ser Asp Asn Ser Gly Thr His Ala Glu Cys Val Thr Asn Asn Ile
   305                 310                 315                 320
   Asp Ala Ala Phe Ala Pro Leu Ala Thr Trp Leu Arg Ala Asn Gly Arg
                   325                 330                 335
   Gln Ala Ile Leu Ser Glu Thr Gly Gly Gly Asn Thr Ala Ser Cys Gln
               340                 345                 350
   Thr Tyr Leu Cys Gln Gln Val Ala Tyr Leu Asn Ala Asn Ser Asp Val
           355                 360                 365
   Tyr Leu Gly Tyr Ile Gly Trp Ser Ala Gly Ser Phe Asp Ser Thr Tyr
       370                 375                 380
   Ile Leu Thr Glu Thr Pro Asn Gly Ser Gly Ser Ser Met Thr Asp Gln
   385                 390                 395                 400
   Ala Leu Val Ala Ala Cys Leu Thr Arg Thr Ser
                   405                 410
<210> 37
   <211> 1242
   <212> DNA
   <213> Sclerotinia sclerotiorum
<400> 37
   atgaaggtgc caactcctct gtacactatc cttccgctag tatccagcgc cacagcacag      60
   ggcgctgcct atgcacagtg tggaggtaaa ggttggacgg gagcaacgac ttgtgttgga     120
   ggctatgtgt gtacttattc gagtgaatat tattcgcaat gtttaccggg aactgcgact     180
   ctaaccactg ttaccagttc gtccaaacca tcaagctcta gtaccaagac ctcatcaagc     240
   gcagcttcaa gctcaacggg aaaaacgaaa tatattggca ccaacatcgc gggttttgac     300
   tttggctgta ccacagatgg cacttgcata acctcggaga tctatcctcc gttgagcagc     360
   atagccaacc atcccgatgg cctcggccag atgtcccact tcgtcaaaag cacagggcac     420
   aatattttcc gcttacctgt cggatggcag tacctcgtca acaacgtttt gggcggcaca     480
   cttgattcca acaatttcgc aacctatgat tcacttgttc aggggtgcct ggcaacaggc     540
   gcaagttgca tcattgacat ccacaattat gctcgatgga acggtgggat cattggtcag     600
   ggcggcccta ctaatgctca attcgtgagc ctttggactc agttggcaaa taagtacaag     660
   gggaatgcga aggtgatttt cggcttgatg aatgagcccc acgacatgcc aaacatcacc     720
   acctgggctg cctcagtcca agcagttgta accgcaatcc gccaagctgg tgctacgtcg     780
   accacgcttc tcttgcctgg aaatgattac acctctgctg ggtctttcat atccgatggc     840
   agtgcagccg ccctgtctaa agtcacgaac ccggatggga caatcacgaa tctgattttt     900
   gacgtgcaca aatacttgga ctcagacaac tccggtacta atcttgaatg tgtgacaaat     960
   aacattgaca ccgcctttgc accgcttgcc acttggctcc gagcaaacgg acgccaggct    1020
   atcctgacag aaaccggtgg tggcaacgtt gcttcctgcg agacatattt gtgccaggaa    1080
   gttgcttatc tcaacgccaa ctcagatgtc tatcttggct atgttggttg ggctgccgga    1140
   tcatttgata cgaattatac actgacggaa acaccgaatg gcagtggttc atcaatgacg    1200
   gaccaaccat tggtcgcggc ttgtctaact agatcgaatt ag                       1242
<210> 38
   <211> 413
   <212> PRT
   <213> Sclerotinia sclerotiorum
<400> 38
   Met Lys Val Pro Thr Pro Leu Tyr Thr Ile Leu Pro Leu Val Ser Ser
   1               5                   10                  15
   Ala Thr Ala Gln Gly Ala Ala Tyr Ala Gln Cys Gly Gly Lys Gly Trp
               20                  25                  30
   Thr Gly Ala Thr Thr Cys Val Gly Gly Tyr Val Cys Thr Tyr Ser Ser
           35                  40                  45
   Glu Tyr Tyr Ser Gln Cys Leu Pro Gly Thr Ala Thr Leu Thr Thr Val
       50                  55                  60
   Thr Ser Ser Ser Lys Pro Ser Ser Ser Ser Thr Lys Thr Ser Ser Ser
   65                  70                  75                  80
   Ala Ala Ser Ser Ser Thr Gly Lys Thr Lys Tyr Ile Gly Thr Asn Ile
                   85                  90                  95
   Ala Gly Phe Asp Phe Gly Cys Thr Thr Asp Gly Thr Cys Ile Thr Ser
               100                 105                 110
   Glu Ile Tyr Pro Pro Leu Ser Ser Ile Ala Asn His Pro Asp Gly Leu
           115                 120                 125
   Gly Gln Met Ser His Phe Val Lys Ser Thr Gly His Asn Ile Phe Arg
       130                 135                 140
   Leu Pro Val Gly Trp Gln Tyr Leu Val Asn Asn Val Leu Gly Gly Thr
   145                 150                 155                 160
   Leu Asp Ser Asn Asn Phe Ala Thr Tyr Asp Ser Leu Val Gln Gly Cys
                   165                 170                 175
   Leu Ala Thr Gly Ala Ser Cys Ile Ile Asp Ile His Asn Tyr Ala Arg
               180                 185                 190
   Trp Asn Gly Gly Ile Ile Gly Gln Gly Gly Pro Thr Asn Ala Gln Phe
           195                 200                 205
   Val Ser Leu Trp Thr Gln Leu Ala Asn Lys Tyr Lys Gly Asn Ala Lys
       210                 215                 220
   Val Ile Phe Gly Leu Met Asn Glu Pro His Asp Met Pro Asn Ile Thr
   225                 230                 235                 240
   Thr Trp Ala Ala Ser Val Gln Ala Val Val Thr Ala Ile Arg Gln Ala
                   245                 250                 255
   Gly Ala Thr Ser Thr Thr Leu Leu Leu Pro Gly Asn Asp Tyr Thr Ser
               260                 265                 270
   Ala Gly Ser Phe Ile Ser Asp Gly Ser Ala Ala Ala Leu Ser Lys Val
           275                 280                 285
   Thr Asn Pro Asp Gly Thr Ile Thr Asn Leu Ile Phe Asp Val His Lys
       290                 295                 300
   Tyr Leu Asp Ser Asp Asn Ser Gly Thr Asn Leu Glu Cys Val Thr Asn
   305                 310                 315                 320
   Asn Ile Asp Thr Ala Phe Ala Pro Leu Ala Thr Trp Leu Arg Ala Asn
                   325                 330                 335
   Gly Arg Gln Ala Ile Leu Thr Glu Thr Gly Gly Gly Asn Val Ala Ser
               340                 345                 350
   Cys Glu Thr Tyr Leu Cys Gln Glu Val Ala Tyr Leu Asn Ala Asn Ser
           355                 360                 365
   Asp Val Tyr Leu Gly Tyr Val Gly Trp Ala Ala Gly Ser Phe Asp Thr
       370                 375                 380
   Asn Tyr Thr Leu Thr Glu Thr Pro Asn Gly Ser Gly Ser Ser Met Thr
   385                 390                 395                 400
   Asp Gln Pro Leu Val Ala Ala Cys Leu Thr Arg Ser Asn
                   405                 410

## Description

La possibilité de produire de l'éthanol à partir de la cellulose a reçu beaucoup d'attention en raison de la disponibilité de grandes quantités de matière première ainsi que de l'intérêt de l'éthanol à titre de carburant. Les matières premières naturelles cellulosiques pour un tel processus sont désignées par le terme "biomasse". De nombreux types de biomasse, par exemple le bois, les résidus agricoles, les cultures herbacées et les déchets solides municipaux, ont été considérés comme des matières premières potentielles pour la production de biocarburant. Ces matières sont constituées principalement de cellulose, d'hémicellulose et de lignine.

La cellulose est un polymère constitué de molécules de glucose reliées par des liens beta 1-4, qui sont très résistants à la dégradation ou à la dépolymérisation. Une fois la cellulose convertie en glucose, celui-ci est facilement fermenté en biocarburant, par exemple l'éthanol, en utilisant une levure.

Les plus anciennes méthodes étudiées pour convertir la cellulose en glucose sont basées sur l'hydrolyse acide. Ce processus peut se faire en présence d'acides concentrés ou dilués. Cependant, plusieurs inconvénients tels que la mauvaise récupération de l'acide lors de l'utilisation d'acides concentrés et la faible production de glucose dans le cadre de l'utilisation d'acides dilués nuisent à l'économie du processus d'hydrolyse acide.

Pour surmonter les inconvénients du processus d'hydrolyse acide, les processus de conversion de la cellulose ont porté plus récemment sur l'hydrolyse enzymatique, à l'aide d'enzymes de type cellulase. Cette hydrolyse enzymatique de la biomasse lignocellulosique (par exemple, la cellulose) présente cependant l'inconvénient d'être un procédé industriel coûteux. De ce fait, il est nécessaire d'utiliser des souches de microorganismes sécréteurs de cellulases de plus en plus performantes. À ce titre, beaucoup de microorganismes comportent des enzymes qui hydrolysent la cellulose, tels que les champignons *Trichoderma, Aspergillus, Humicola, Fusarium* ainsi que les bactéries telles que *Thermomonospora, Bacillus, Cellulomouas* et *Streptomyces.* Les enzymes sécrétées par ces microorganismes possèdent trois types d'activités utiles dans la conversion de la cellulose en glucose et se divisent en trois groupes: les endoglucanases, qui attaquent les fibres de celluloses aléatoirement en interne, les exoglucanases qui vont attaquer les extrémités des fibres en libérant du cellobiose, et les β-glucosidases qui vont hydrolyser ce cellobiose en glucose. D'autres classes d'enzymes telles que les hémicellulases ou la classe d'enzymes récemment découverte des polysaccharides mono-oxygénases peuvent jouer également un rôle dans l'efficacité de l'hydrolyse.

Il y a un intérêt industriel fort pour la diminution du coût de l'hydrolyse enzymatique, et cette diminution passe par l'utilisation d'une dose réduite d'enzymes et donc des cocktails d'enzymes plus efficaces. En conséquence, plusieurs demandes de brevets décrivent des enzymes naturelles aux capacités supérieures à celles de *Trichoderma reesei,* ou des variants améliorés par génie génétique. On peut citer les demandes de brevet US2010304464, WO2010066411 et WO2013029176 concernant les exoglucanases, les demandes WO2007109441, WO2012149192, WO2008088724, WO201203681 WO2013096244 et WO2010076388 concernant les endoglucanases, les demandes WO2010029259, WO2010135836 ou WO2010022518 concernant les β-glucosidases, ou encore les demandes WO12135659, WO12149344 concernant les polysaccharides mono-oxygénases.

La demande WO2011/153516 décrit également des souches de levures exprimant des enzymes saccharolytiques.

Les enzymes hydrolysant la biomasse lignocellulosique sont classées dans le système CAZy (Cantarel, B. L., Coutinho, P. M., Rancurel, C., Bernard, T., Lombard, V., & Henrissat, B. (2009). The Carbohydrate-Active EnZymes database (CAZy): an expert resource for Glycogenomics. Nucleic acids research, 37, D233-8) sur des critères principalement structuraux. Les endoglucanases peuvent appartenir aux familles GH 5, 6, 7, 8, 9, 12, 16, 18, 19, 26, 44, 45, 48, 51, 74 et 124.

Pour qu'une hydrolyse de la biomasse lignocellulosique soit efficace et économiquement rentable, le mélange enzymatique doit comporter des proportions équilibrées d'activités enzymatiques diverses (entre autres, mais non exclusivement exoglucanases, endoglucanases, xylanases et β-glucosidases). A titre d'exemple, dans les mélanges natifs de *Trichoderma reesei* on constate généralement la présence de 60-70% d'exoglucanases, 15-20% d'endoglucanases, quelques pourcentages d'hémicellulases et environ 5-10% de β-glucosidase. Ce mélange convient pour hydrolyser la majorité des substrats prétraités (ex. type paille de blé explosée à la vapeur en conditions acides) avec des rendements acceptables. En somme, l'augmentation de l'activité endoglucanase ne doit pas se faire au détriment des autres activités enzymatiques. Les spécificités fonctionnelles de ces enzymes sont aujourd'hui mal connues. Le génome de *Trichoderma reesei* comporte au moins 3 enzymes principales, issues des familles 7 (EG1, cel7b), 5 (EG2, cel5a) et 12 (EG3, Cel12a). Les enzymes EG1 et EG2 sont les endoglucanases majoritaires et peuvent représenter jusqu'à 10-20% en masse du cocktail d'enzymes complet produit par *T. reesei.*

Les endoglucanases (EC 3.2.1.4), premières enzymes à agir sur la cellulose, sont connues pour avoir un rôle majeur dans l'hydrolyse en augmentant le nombre de sites que peuvent attaquer les exoglucanases tout en diminuant le degré de polymérisation des microfibrilles attaquées. De récents travaux (Szijártó, N., Siika-aho, M., Sontag-Strohm, T., & Viikari, L. (2011). Liquefaction of hydrothermally pretreated wheat straw at high-solids content by purified Trichoderma enzymes. Bioresource technology, 102(2), 1968-74) soulignent leur rôle dans la diminution de la viscosité de la biomasse au cours des premières heures de l'hydrolyse. Cette diminution de viscosité peut avoir un impact très important sur les coûts opératoires du procédé.

Le problème de viscosité est exacerbé dans le cas de procédés imposant le recours à une basse température telle que la saccharification simultanée à la fermentation (SSF), qui met en jeu à la fois les enzymes hydrolysant la biomasse et le micro-organisme qui convertit les monomères de sucres en éthanol.

L'hydrolyse et la fermentation peuvent être réalisées suivant différents schémas. Le plus courant consiste en une hydrolyse et une fermentation séparées (SHF-Separate Hydrolysis and Fermentation). Cette méthode permet d'optimiser chaque étape par le maintien des conditions optimales de réaction. Cette fermentation s'effectue de manière extemporanée, à une température comprise entre environ 28°C et environ 30°C, tandis que l'hydrolyse a lieu généralement à une température d'au moins 45°C. Cependant, en SHF, les sucres libérés en fin de réaction sont présents à très forte concentration et entraînent une inhibition des enzymes, ralentissant l'efficacité du procédé. Pour éviter ces inconvénients, un autre type de procédé peut être envisagé. En SSF, les deux étapes (hydrolyse et fermentation des hexoses) ont lieu de manière simultanée, empêchant l'accumulation des sucres à des concentrations inhibitrices pour les enzymes. Les coûts d'investissement sont également réduits grâce à l'utilisation d'un seul réacteur. Le taux d'hydrolyse est plus élevé suite à l'absence d'inhibition car les sucres libérés sont utilisés immédiatement pour la fermentation en éthanol. Dans cette méthode, la température du réacteur constitue nécessairement un compromis entre les températures optimales d'hydrolyse et de fermentation, typiquement entre environ 30°C et environ 35°C. Cependant, à une telle température, l'activité des enzymes cellulolytiques est diminuée de 30% environ.

La SSF permet également l'expression d'enzymes dégradant la cellulose dans l'organisme fermentant les sucres, ce qui permet de limiter, ou dans un cas extrême de supprimer le recours aux enzymes produites lors d'une étape séparée.

En conséquence, l'obtention d'enzymes maintenant une activité endoglucanase efficace aux températures optimales d'hydrolyse et de fermentation (soit entre 30°C et 50°C) tout en gardant la proportion de l'ensemble des enzymes du mélange serait un gain significatif pour le procédé de conversion de biomasse lignocellulosique en biocarburant.

### Description

Les inventeurs ont développé un polypeptide ayant une activité endoglucanase améliorée, notamment par rapport à l'activité endoglucanase de la protéine sauvage EG2 de séquence SEQ ID NO :2. EG2 correspond à l'endoglucanase 2 de *Trichoderma reesei.*

Dans cette optique, les déposants, ont eu le grand mérite de trouver, après de nombreuses recherches, un polypeptide isolé ou purifié ayant une activité endoglucanase améliorée par rapport à l'activité endoglucanase de la protéine sauvage EG2 (SEQ ID NO :2).

Selon la description, le polypeptide est choisi dans le groupe consistant en :
i) une séquence d'acides aminés choisie parmi SEQ ID NO :4, SEQ ID NO :6, SEQ ID NO :8, SEQ ID NO :10, SEQ ID NO :12; SEQ ID NO :14 SEQ ID NO :16, SEQ ID NO :18, SEQ ID NO :20, SEQ ID NO :22, SEQ ID NO :24, SEQ ID NO :26, SEQ ID NO :28, SEQ ID NO :30, SEQ ID NO :32 et SEQ ID NO :34;
ii) une séquence d'acides aminés présentant un pourcentage d'identité d'au moins 70%, préférentiellement 75%, 80%, 85%, 90%, 95%, 98% ou 99%, par rapport à la séquence SEQ ID NO :4, SEQ ID NO :6, SEQ ID NO :8, SEQ ID NO :10, SEQ ID NO :12; SEQ ID NO :14 SEQ ID NO :16, SEQ ID NO :18, SEQ ID NO :20, SEQ ID NO :22, SEQ ID NO :24, SEQ ID NO :26, SEQ ID NO :28, SEQ ID NO :30, SEQ ID NO :32 ou SEQ ID NO :34.

L'invention est définie dans les revendications.

Le pourcentage d'identité d'une séquence donnée par rapport à SEQ ID NO :4, SEQ ID NO :6, SEQ ID NO :8, SEQ ID NO :10, SEQ ID NO :12; SEQ ID NO :14 SEQ ID NO :16, SEQ ID NO :18, SEQ ID NO :20, SEQ ID NO :22, SEQ ID NO :24, SEQ ID NO :26, SEQ ID NO :28, SEQ ID NO :30, SEQ ID NO :32 ou SEQ ID NO :34, correspond au nombre de résidus identiques entre cette séquence donnée et SEQ ID NO :4, SEQ ID NO :6, SEQ ID NO :8, SEQ ID NO :10, SEQ ID NO :12; SEQ ID NO :14 SEQ ID NO :16, SEQ ID NO :18, SEQ ID NO :20, SEQ ID NO :22, SEQ ID NO :24, SEQ ID NO :26, SEQ ID NO :28, SEQ ID NO :30, SEQ ID NO :32 ou SEQ ID NO :34 divisé par le nombre de résidus dans SEQ ID NO :4, SEQ ID NO :6, SEQ ID NO :8, SEQ ID NO :10, SEQ ID NO :12; SEQ ID NO :14 SEQ ID NO :16, SEQ ID NO :18, SEQ ID NO :20, SEQ ID NO :22, SEQ ID NO :24, SEQ ID NO :26, SEQ ID NO :28, SEQ ID NO :30, SEQ ID NO :32 ou SEQ ID NO :34. Lorsqu'on utilise la base de données GenomeQuest, ledit pourcentage d'identité par rapport à SEQ ID NO :4, SEQ ID NO :6, SEQ ID NO :8, SEQ ID NO :10, SEQ ID NO :12; SEQ ID NO :14 SEQ ID NO :16, SEQ ID NO :18, SEQ ID NO :20, SEQ ID NO :22, SEQ ID NO :24, SEQ ID NO :26, SEQ ID NO :28, SEQ ID NO :30, SEQ ID NO :32 ou SEQ ID NO :34 correspond au pourcentage d'identité d'Interrogation (% id Query), où Interrogation correspond à la séquence SEQ ID NO :4, SEQ ID NO :6, SEQ ID NO :8, SEQ ID NO :10, SEQ ID NO :12; SEQ ID NO :14 SEQ ID NO :16, SEQ ID NO :18, SEQ ID NO :20, SEQ ID NO :22, SEQ ID NO :24, SEQ ID NO :26, SEQ ID NO :28, SEQ ID NO :30, SEQ ID NO :32 ou SEQ ID NO :34.

Dans un autre mode de réalisation, le polypeptide de l'invention tel que décrit précédemment est caractérisé en ce que son expression dans un organisme fermentaire est au moins égale à l'expression de la protéine sauvage EG2 (SEQ ID NO :2).

L'homme du métier pourra par exemple déterminer l'augmentation ou autrement dit l'amélioration de l'activité enzymatique soit à l'aide du substrat Carboxy-Méthyl-cellulose (CMC), soit avec un substrat chromogénique (p-Nitrophenyl glycoside). L'activité enzymatique sera respectivement révélée par dosage colorimétrique des sucres réducteurs ou bien du nitrophénol libérés.

De préférence, le polypeptide de l'invention a une activité enzymatique améliorée d'au moins 10%, préférentiellement d'au moins 20%, préférentiellement d'au moins 30%, par rapport à l'activité endoglucanase de la protéine EG2 de séquence d'acides aminés SEQ ID NO :2.

Un exemple de protocole, que l'homme du métier pourra utiliser pour déterminer si un polypeptide selon l'invention présente une activité enzymatique améliorée par rapport à celle de la protéine sauvage EG2 (SEQ ID NO :2), est le suivant :
- formation d'une culture stock d'E. *coli* exprimant un polypeptide selon l'invention pendant toute la nuit à 37°C ;
- ensemencement d'un milieu de culture LB avec 1% de culture stock à 37°C jusqu'à l'obtention d'une densité optique de 0.4 ;
- culture desdites cellules à 20°C pendant 18h ;
- centrifugation pendant 5 minutes à 7900 rpm ;
- resuspension des culots cellulaires avec du tampon citrate phosphate 100 mM à pH 5 contenant 1 mg/mL de lysozyme (DO₆₀₀ finale 100);
- incubation des cellules resuspendues 30 minutes sur la glace ;
- lyse des cellules par 3 cycles de congélation/décongélation ;
- fractionnement de l'ADN par sonication ;
- centrifugation 30 minutes à 13000 rpm ;
- incubation de 100 µL de surnageant de cassage dilué 50 fois avec 100 µL de tampon citrate phosphate 100 mM à pH 5 contenant 1% de CMC pendant 1h à 35 et 50°C ;
- prélèvement de 100 µL de réaction ;
- ajout de 100 µL de réactif DNS (Miller, 1959);
- incubation 5 minutes à 100°C ;
- incubation 3 minutes sur la glace ;
- centrifugation pendant 10 minutes à 3000 rpm ;
- lecture de la densité optique à 540 nm sur 150 µL de surnageant.

L'invention a également pour objet, un acide nucléique purifié ou isolé codant au moins un polypeptide selon l'invention tel que décrit précédemment.

Le TABLEAU 1 ci-dessous comprend les identifications des séquences nucléiques et peptidiques pour les gènes EG2, les gènes des endoglucanases 2 putative de *Botryotinia fuckeliana* (gène BF) et de l'endoglucanase 2 putative de *Sclerotinia sclerotiorum* (gène SS), ainsi que pour les séquences nucléiques et polypeptides de l'invention et de la description.

Selon la description, ledit acide nucléique purifié ou isolé peut être choisi parmi les séquences suivantes: SEQ ID NO :3, SEQ ID NO :5, SEQ ID NO :7, SEQ ID NO :9, SEQ ID NO :11; SEQ ID NO :13, SEQ ID NO :15, SEQ ID NO :17, SEQ ID NO :19, SEQ ID NO :21, SEQ ID NO :23, SEQ ID NO :25, SEQ ID NO :27, SEQ ID NO :29, SEQ ID NO :31 et SEQ ID NO :33.

**TABLEAU 1**

| **Clones** | **Acide nucléique** | **Polypeptide** |
|---|---|---|
| EG2 (sauvage) | SEQ ID NO :1 | SEQ ID NO :2 |
| 37D12 | SEQ ID NO :3 | SEQ ID NO :4 |
| 45A7 | SEQ ID NO :5 | SEQ ID NO :6 |
| 46H1 | SEQ ID NO :7 | SEQ ID NO :8 |
| 50F10 | SEQ ID NO :9 | SEQ ID NO :10 |
| 108G5 | SEQ ID NO: 11 | SEQ ID NO :12 |
| 140F7 | SEQ ID NO :13 | SEQ ID NO :14 |
| 146C4 | SEQ ID NO :15 | SEQ ID NO :16 |
| 149E4 | SEQ ID NO :17 | SEQ ID NO :18 |
| 173C6 | SEQ ID NO :19 | SEQ ID NO :20 |
| 191H11 | SEQ ID NO :21 | SEQ ID NO :22 |
| 222E1 | SEQ ID NO :23 | SEQ ID NO :24 |
| 225C7 | SEQ ID NO :25 | SEQ ID NO :26 |
| 227C4 | SEQ ID NO :27 | SEQ ID NO :28 |
| 229D1 | SEQ ID NO :29 | SEQ ID NO :30 |
| 231C9 | SEQ ID NO :31 | SEQ ID NO :32 |
| 330F9 | SEQ ID NO :33 | SEQ ID NO :34 |
| Gène BF | SEQ ID NO :35 | SEQ ID NO :36 |
| Gène SS | SEQ ID NO :37 | SEQ ID NO :38 |

L'invention porte également sur un vecteur comprenant un acide nucléique selon l'invention tel que décrit précédemment.

Selon l'invention, on entend par « vecteur » toute séquence d'ADN dans laquelle il est possible d'insérer des fragments d'acide nucléique étranger, les vecteurs permettant d'introduire de l'ADN étranger dans une cellule hôte. On peut citer de manière non-exhaustive comme vecteurs : les plasmides, les cosmides, les chromosomes artificiels de levures (YAC), les chromosomes artificiels de bactéries (BAC), les chromosomes artificiels dérivés du bactériophage P1 (PAC) ou les vecteurs dérivés de virus.

Selon l'invention, l'acide nucléique tel que décrit précédemment pourra être lié opérationnellement à un promoteur, un terminateur ou toute autre séquence nécessaire à son expression dans la cellule hôte.

Le vecteur selon l'invention pourra également porter un marqueur de sélection. On entend par « marqueur de sélection » un gène dont l'expression confère aux cellules qui le contiennent une caractéristique permettant de les sélectionner. Il s'agit par exemple d'un gène de résistance aux antibiotiques.

L'invention a également pour objet une cellule hôte isolée comprenant soit au moins l'un des polypeptides selon l'invention tels que décrit précédemment, soit au moins l'un des acides nucléiques selon l'invention tels que décrit précédemment ou soit au moins l'un des vecteurs selon l'invention tels que décrit précédemment.

L'homme du métier pourra introduire l'un des polypeptides, l'un des acides nucléiques ou l'un des vecteurs selon l'invention tels que décrit précédemment dans la cellule hôte selon l'invention par des méthodes conventionnelles bien connues. Par exemple, on peut citer le traitement au chlorure de calcium, l'électroporation, l'utilisation d'un pistolet à particules.

Selon un mode de réalisation, l'homme du métier pourra introduire dans la cellule hôte selon l'invention et par des méthodes conventionnelles, plusieurs copies d'un acide nucléique selon l'invention codant un polypeptide selon l'invention ayant une activité endoglucanase améliorée selon l'invention.

Selon un mode de réalisation, la cellule hôte isolée selon l'invention telle que décrite précédemment est choisie parmi *Trichoderma, Aspergillus, Neurospora, Humicola, Myceliophthora, Chrysosporium, Pénicillium, Fusarium, Thermomouospora, Bacillus, Pseudomouas, Escherichia, Clostridium, Cellulomouas, Streptomyces, Yarrowia, Pichia* et *Saccharomyces.*

Selon un mode de réalisation préféré, la cellule hôte isolée selon l'invention telle que décrite précédemment est choisie parmi *Trichoderma reesei, Trichoderma viridae, Trichoderma koningii, Aspergillus niger, Aspergillus nidulans, Aspergillus wentii, Aspergillus oryzae, Aspergillus phoenicis, Myceliophthora thermopila, Chrysosporium luckuowense, Neurospora crassa, Humicola grisae, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* et *Saccharomyces cerevisiae.*

Selon un mode de réalisation préféré, la cellule hôte isolée selon l'invention telle que décrite précédemment est choisie parmi *Trichoderma reesei* et *Saccharomyces cerevisiae.*

L'invention a également pour objet l'utilisation de l'un quelconque des polypeptides selon l'invention décrits précédemment pour l'hydrolyse de la cellulose.

L'invention a également pour objet, l'utilisation de l'un quelconque des polypeptides selon l'invention décrits précédemment pour la production de biocarburant.

Selon l'invention, le terme biocarburant peut être défini comme tout produit issu de la transformation de la biomasse et pouvant être utilisé à des fins énergétiques. D'une part et sans vouloir se limiter, on peut citer à titre d'exemple des biogaz, des produits pouvant être incorporés (éventuellement après transformation ultérieure) à un carburant ou être un carburant à part entière, tels que des alcools (l'éthanol, le butanol et/ou l'isopropanol selon le type d'organisme fermentaire utilisé), des solvants (acétone), des acides (butyrique), des lipides et leurs dérivés (acides gras à courtes ou longues chaînes, esters d'acides gras), ainsi que l'hydrogène.

De manière préférée, le biocarburant selon l'invention est un alcool, par exemple l'éthanol, le butanol et/ou l'isopropanol. Plus préférentiellement, le biocarburant selon l'invention est l'éthanol.

Dans un autre mode de réalisation, le biocarburant est du biogaz.

Dans un autre mode de réalisation, le produit est une molécule intéressant l'industrie chimique, comme par exemple, un autre alcool tel que le 1,2-propane diol, le 1,3-propane diol, le 1,4-butane diol, le 2,3-butane diol, des acides organiques comme l'acide acétique, propionique, acrylique, butyrique, succinique, malique, fumarique, citrique, itaconique, ou des hydroxyacides comme l'acide glycolique, hydroxypropionique, ou lactique.

On décrit ci-dessous un mode de réalisation de production d'un cocktail enzymatique utile pour l'hydrolyse de la lignocellulose. Les souches de champignons filamenteux, de préférence *Trichoderma,* plus préférentiellement *T. reesei,* capables d'exprimer au moins un polypeptide selon l'invention sont cultivées en fermenteurs, en présence d'un substrat carboné, tel que le lactose ou le glucose, choisi pour la croissance du microorganisme. Dans un mode de réalisation, ce substrat carboné, selon sa nature, est introduit dans le fermenteur avant stérilisation ou est stérilisé séparément et introduit dans le fermenteur après stérilisation de ce dernier pour obtenir une concentration initiale de 20 à 35 g/L.

Une solution aqueuse contenant le substrat choisi pour la production des enzymes est ensuite ajoutée. Une composition enzymatique agissant sur la biomasse lignocellulosique produite par les champignons est enfin récupérée par filtration du milieu de culture. Dans cette composition, on retrouve notamment, la β-glucosidase, l'exoglucanase et l'endoglucanase selon l'invention. Dans un mode de réalisation, la solution aqueuse contenant le substrat choisi pour la production des enzymes est préparée à la concentration de 200-250 g/L et contient en outre un substrat inducteur tel que le lactose. Cette solution aqueuse est injectée après l'épuisement du substrat carboné initial de façon à apporter une quantité optimisée, comprise entre 35 et 45 mg/g de cellules ("fed batch"). Pendant cette phase de "fed batch", la concentration résiduelle en sucre dans le milieu de culture est inférieure à 1 g/L et les enzymes agissant sur la biomasse lignocellulosique sont sécrétées par le champignon. Ces dernières peuvent être récupérées par filtration du milieu de culture.

L'invention a pour objet une composition enzymatique apte à agir sur la biomasse lignocellulosique, ladite composition enzymatique étant produite de préférence par des champignons filamenteux et comprend au moins un polypeptide ayant une activité endoglucanase améliorée par rapport à l'activité endoglucanase de la protéine sauvage EG2. Par « champignons filamenteux », on entend notamment *Trichoderma,* plus préférentiellement *T*. *reesei.*

Enfin, l'invention a pour objet un procédé de production de biocarburant à partir de la biomasse comprenant les étapes successives suivantes :
- mise en suspension en phase aqueuse de la biomasse à hydrolyser;
- hydrolyse en présence d'une composition enzymatique de la biomasse lignocellulosique telle que décrite précédemment de manière à produire un hydrolysat contenant du glucose;
- fermentation du glucose de l'hydrolysat de manière à produire un moût de fermentation;
- séparation du biocarburant du moût de fermentation.

Dans un mode de réalisation, la biomasse à hydrolyser est mise en suspension en phase aqueuse à raison de 6 à 40 % de matière sèche, de préférence 20 à 30 %. Le pH est ajusté entre 4 et 5,5; de préférence entre 4,8 et 5,2 et la température entre 40 et 60°C, de préférence entre 45 et 50°C. La réaction d'hydrolyse est démarrée par l'ajout de la composition enzymatique agissant sur la biomasse lignocellulosique; la quantité habituellement utilisée est de 10 à 30 mg de protéines excrétées par gramme de substrat prétraité ou moins. La réaction dure généralement de 15 à 48 heures. La réaction est suivie par dosage des sucres libérés, notamment le glucose. La solution de sucres est séparée de la fraction solide non hydrolysée, essentiellement constituée de lignine, par filtration ou centrifugation et est ensuite traitée dans une unité de fermentation.

Dans un mode de réalisation, le biocarburant pourra être séparé du moût de fermentation par exemple par distillation.

Un autre objet de l'invention est un procédé de production de biocarburant à partir de la biomasse, caractérisé en ce qu'il comprend les étapes successives suivantes :
- mise en suspension en phase aqueuse de la biomasse à hydrolyser;
- ajout simultané d'une composition enzymatique telle que définie précédemment et d'un organisme fermentaire, de préférence à une température comprise entre 30°C et 35°C, de manière à produire un moût de fermentation;
- séparation du biocarburant du moût de fermentation.

De préférence, la composition enzymatique et l'organisme fermentaire sont ajoutés simultanément puis incubés à une température comprise entre 30°C et 35°C pour produire un moût de fermentation.

Selon ce mode de réalisation, la cellulose présente dans la biomasse est convertie en glucose, et en même temps, dans le même réacteur, l'organisme fermentaire (par exemple une levure) convertit le glucose en produit final selon un procédé de SSF (Simultaneous Saccharification and Fermentation) connu de l'homme du métier. Selon les capacités métaboliques et hydrolytiques de l'organisme fermentaire, le bon déroulement de l'opération peut nécessiter l'addition d'une quantité plus ou moins importante de mélange cellulolytique exogène.

Dans un autre mode de réalisation, l'organisme fermentaire produit également le polypeptide objet de l'invention par sécrétion ou en surface de sa cellule, éventuellement conjointement à d'autres enzymes agissant sur la biomasse lignocellulosique, limitant ou supprimant ainsi le besoin en enzymes produites par le champignon filamenteux. De préférence, l'organisme fermentaire est une cellule hôte telle que décrite précédemment.

Ainsi, de préférence, l'invention a pour objet un procédé de production de biocarburant à partir de la biomasse, comprenant les étapes successives suivantes :
- mise en suspension en phase aqueuse de la biomasse à hydrolyser ;
- ajout d'une ou plusieurs cellules hôtes telles que décrites précédemment, avec un organisme fermentaire et/ou une composition enzymatique telle que décrite précédemment, de manière à produire un moût de fermentation ;
- séparation du biocarburant du moût de fermentation.

De préférence, les cellules hôtes avec la composition enzymatique et/ou l'organisme fermentaire, sont ajoutées puis incubés à une température comprise entre 30°C et 35°C pour produire un moût de fermentation.

L'utilisation du polypeptide selon l'invention présentant une meilleure activité endoglucanase selon la présente invention présente ainsi l'avantage d'obtenir un meilleur rendement de production de glucose. Ainsi la présente invention permet d'utiliser moins d'enzyme qu'auparavant, ce qui présente un avantage du point de vue économique.

D'autres aspects, objets, avantages et caractéristiques de l'invention, seront présentés à la lecture de la description non restrictive qui suit et qui décrit des modes de réalisation préférés de l'invention donnés par le biais d'exemples et des FIGURES.

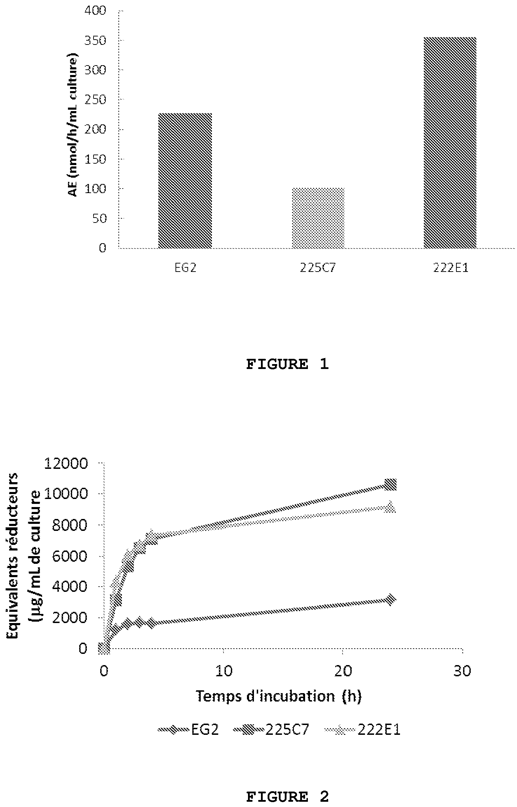

La FIGURE 1 est un graphique représentant l'hydrolyse du *p*-nitrophenyl-β-cellotrioside (pNPC3) par l'endoglucanase de référence EG2 (SEQ ID NO :2) et des mutants 222E1 et 225C7 (respectivement SEQ ID NO :24 et 26) sécrétés dans le milieu de culture des souches Scα-EG2 et Scα-222E1 et Scα-225C7 respectivement.
La FIGURE 2 est un graphique représentant l'hydrolyse de la CMC 1% par l'endoglucanase de référence EG2 (SEQ ID : 2) et des mutants 222E1 et 225C7 (respectivement SEQ ID NO :24 et 26) sécrétés dans le milieu de culture des souches Scα-EG2 et Scα-222E1 et Scα-225C7 respectivement.

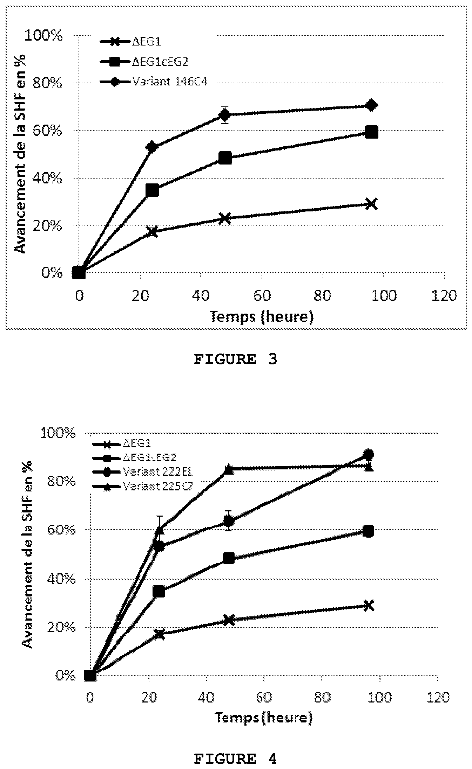

La FIGURE 3 est un graphique présentant les résultats de SHF pour le cocktail issu de la souche 146C4/7, un cocktail de référence produit par la souche CL847 ΔEG1 (ΔEG1) supplémenté en β-glucosidase et un autre cocktail de référence produit par la souche CL847 ΔEG1 retransformée avec le gène de référence EG2 (ΔEG1cEG2) supplémenté en β-glucosidase.
La FIGURE 4 est un graphique présentant les résultats de SHF pour le cocktail issu de la souche 222E1/1 (SEQ ID NO :24) et le cocktail issu de la souche 225C7/7 (SEQ ID NO :26), un cocktail de référence produit par la souche CL847 ΔEG1 (ΔEG1) supplémenté en β-glucosidase et un autre cocktail de référence produit par la souche CL847 ΔEG1 retransformée avec le gène de référence EG2 (ΔEG1cEG2) supplémenté en β-glucosidase.

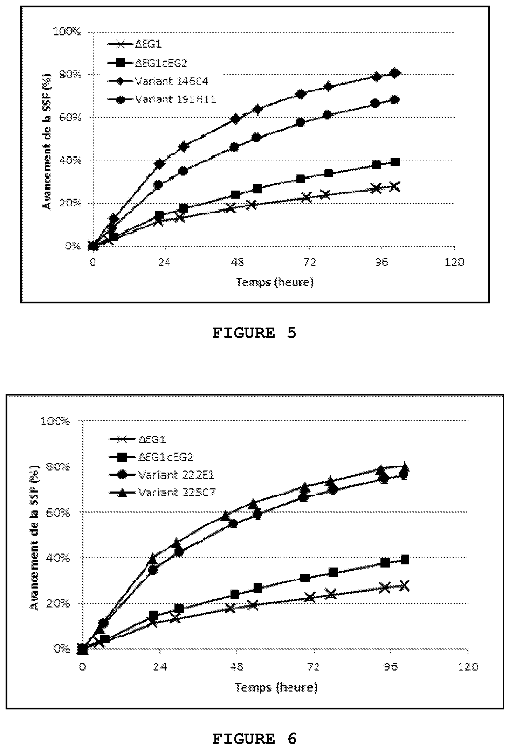

La FIGURE 5 est un graphique présentant les résultats de SSF pour le cocktail issu de la souche 146C4/7 (SEQ ID NO :16) et pour le cocktail issu de la souche 191H11/9 (SEQ ID NO 22), un cocktail de référence produit par la souche CL847 ΔEG1 (ΔEG1) supplémenté en β-glucosidase et un autre cocktail de référence produit par la souche CL847 ΔEG1 retransformée avec le gène de référence EG2 (ΔEG1cEG2) supplémenté en β-glucosidase.
La FIGURE 6 est un graphique présentant la moyenne des résultats de SSF pour les 3 cocktails issus des souches 222E1/1, 222E1/2 et 222E1/7 (SEQ ID NO :24) et les résultats pour le cocktail issu de la souche 225C7/7 (SEQ ID NO :26), un cocktail de référence produit par la souche CL847 ΔEG1 (ΔEG1) supplémenté en β-glucosidase et un autre cocktail de référence produit par la souche CL847 ΔEG1 retransformée avec le gène de référence EG2 (ΔEG1cEG2) supplémenté en β-glucosidase.

### EXEMPLES

### EXEMPLE 1 : évolution par L-shuffling

La séquence du gène de l'endoglucanase 2 (EG2) de *Trichoderma reesei* a été soumise à un tour de L-shuffling selon le procédé breveté décrit dans EP1104457B1 avec les gènes de l'endoglucanase 2 putative de *Botryotinia fuckeliana* (gène BF) et de l'endoglucanase 2 putative de *Sclerotinia sclerotiorum* (gène SS) présentant chacune 64% d'identité avec le gène de référence de EG2 (SEQ ID NO :1).

### 1-Criblage à haut débit

Un test de criblage à haut débit a été mis au point afin de sélectionner les meilleurs clones issus du L-shuffling, c'est-à-dire ceux présentant au moins 20% d'amélioration de l'activité endoglucanase par rapport à l'enzyme de *T. reesei.*

Le test de criblage à haut débit a été réalisé selon les étapes suivantes :
- isolement sur gélose des clones *de E. coli* exprimant les variants de l'enzyme recombinante selon l'invention et mise en pré-cultures en milieu LB desdites colonies pendant la nuit à 37°C ;
- inoculation d'un milieu LB à 6% avec la pré-culture puis incubation 5 h à 37°C puis 17 h à 20°C;
- centrifugation 10 minutes à 3000 rpm ;
- lyse des cellules par ajout de 80 µL d'une solution de lysozyme à 1 mg/mL dans du tampon citrate phosphate 0.1 M à pH 5 ;
- incubation pendant 4 h à température ambiante ;
- ajout de 80 µL de tampon citrate phosphate 0.1M pH 5 contenant 1% de carboxy-méthyl cellulose ;
- incubation 3 h à 35°C ;
- centrifugation pendant 10 minutes à 3000 rpm ;
- prélèvement de 100 µL de surnageant ;
- ajout de 100 µL de réactif DNS ;
- incubation 10 minutes à 100°C puis 5 minutes sur la glace ;
- lecture de la DO à 540 nm sur 120 µL.

Dans ces conditions de criblage, une amélioration de l'activité endoglucanase (augmentation de la DO à 540 nm) par rapport à l'enzyme de référence EG2 (SEQ ID NO :2) a été trouvée dans les clones 37D12, 45A7, 46H1, 50F10, 108G5, 140F7, 146C4, 149E4, 173C6, 191H11, 222E1, 225C7, 227C4, 229D1, 231C9 et 330F9.

### 2-Détermination de l'amélioration de l'activité endoglucanase

### 2-1/ Sur le substrat carboxy-méthyl cellulose (CMC)

Afin de d'estimer le kcat des clones 37D12, 45A7, 46H1, 50F10, 108G5, 140F7, 146C4, 149E4, 173C6, 191H11, 222E1, 225C7, 227C4, 229D1, 231C9 et 330F9 par rapport aux enzymes de référence, on procède de la façon suivante :
- préparation d'une culture stock d'E. *coli* exprimant une enzyme recombinante selon l'invention pendant toute la nuit à 37°C ;
- ensemencement d'un milieu de culture LB avec 1% de culture stock à 37°C jusqu'à l'obtention d'une densité optique à 600 nm de 0.4 ;
- culture desdites cellules à 20°C pendant 18 h ;
- centrifugation pendant 5 minutes à 7900 rpm ;
- resuspension des culots cellulaires avec du tampon citrate phosphate 0.1 M à pH 5 contenant 1 mg/mL de lysozyme (DO₆₀₀ finale 100);
- incubation des cellules resuspendues 30 minutes sur la glace ;
- lyse des cellules par trois cycles de congélation/décongélation ;
- fractionnement de l'ADN par sonication pendant 3 secondes à puissance 5 ;
- centrifugation 30 minutes à 13000 rpm ;
- incubation de 100 µL de surnageant de cassage avec 100 µL de tampon citrate phosphate 0.1 M à pH 5 contenant 1% de CMC pendant 1 heure à 35 et 50°C ;
- prélèvement de 100 µL de réaction ;
- ajout de 100 µL de réactif DNS ;
- incubation 5 minutes à 100°C ;
- incubation 3 minutes sur la glace ;
- centrifugation 10 minutes à 3000 rpm ;
- lecture de la densité optique à 540 nm sur 150 µL.

Selon l'invention, le calcul des kcat est fait de la façon suivante :
- expression des DO à 540 nm en fonction de la quantité de protéine d'intérêt (en nM) ;
- soustraction de la valeur du témoin négatif ;
- division par le coefficient de la gamme étalon de glucose (différentes quantités de glucose sont révélées avec le DNS) ;
- division par le temps de réaction.

Le TABLEAU 2 présente la valeur des kcat ainsi que le facteur d'amélioration par rapport à la protéine de référence EG2 (SEQ ID NO :2) obtenus pour les clones 37D12, 45A7, 46H1, 50F10, 108G5, 140F7, 146C4, 149E4, 173C6, 191H11, 222E1, 225C7, 227C4, 229D1, 231C9 et 330F9 dans les conditions expérimentales du test d'activité sur CMC.

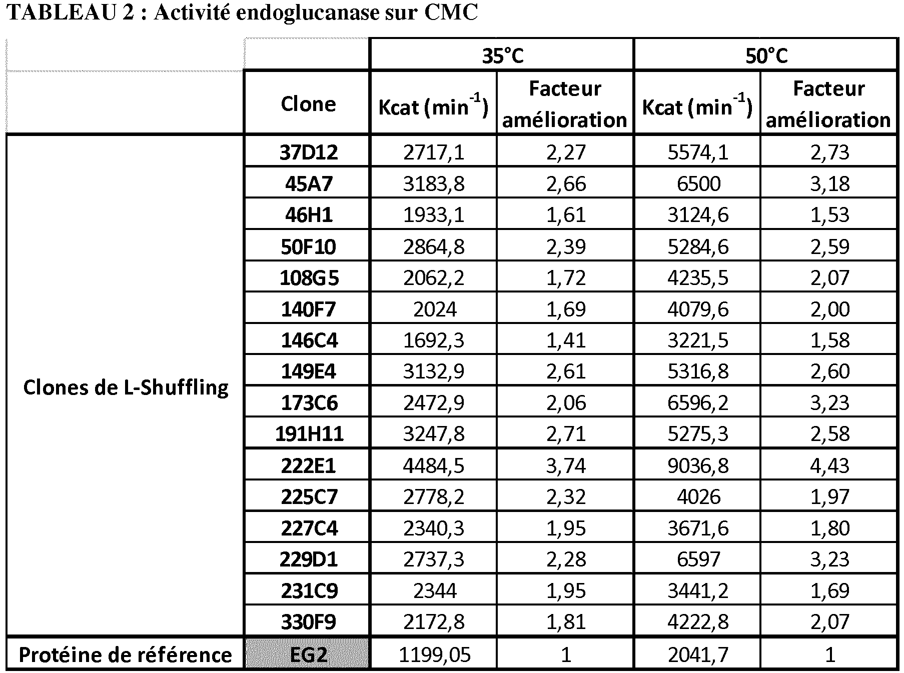

Les résultats montrent des améliorations d'activité enzymatique significatives par rapport à l'enzyme de référence EG2 (SEQ ID NO :2) pour ces clones.

### 2-2/ Sur le substrat phosphoric acid swollen cellulose (PASC)

L'amélioration d'activité des clones 37D12, 45A7, 46H1, 50F10, 108G5, 140F7, 146C4, 149E4, 173C6, 191H11, 222E1, 225C7, 227C4, 229D1, 231C9 et 330F9 a ensuite été confirmée sur un second substrat : phosphoric acid swollen cellulose (PASC).

Le TABLEAU 3 présente la valeur des kcat ainsi que les facteurs d'amélioration obtenus pour les clones 37D12, 45A7, 46H1, 50F10, 108G5, 140F7, 146C4, 149E4, 173C6, 191H11, 222E1, 225C7, 227C4, 229D1, 231C9 et 330F9 à 50°C par rapport à la protéine de référence EG2 (SEQ ID NO :2) dans les conditions expérimentales du test d'activité sur PASC.

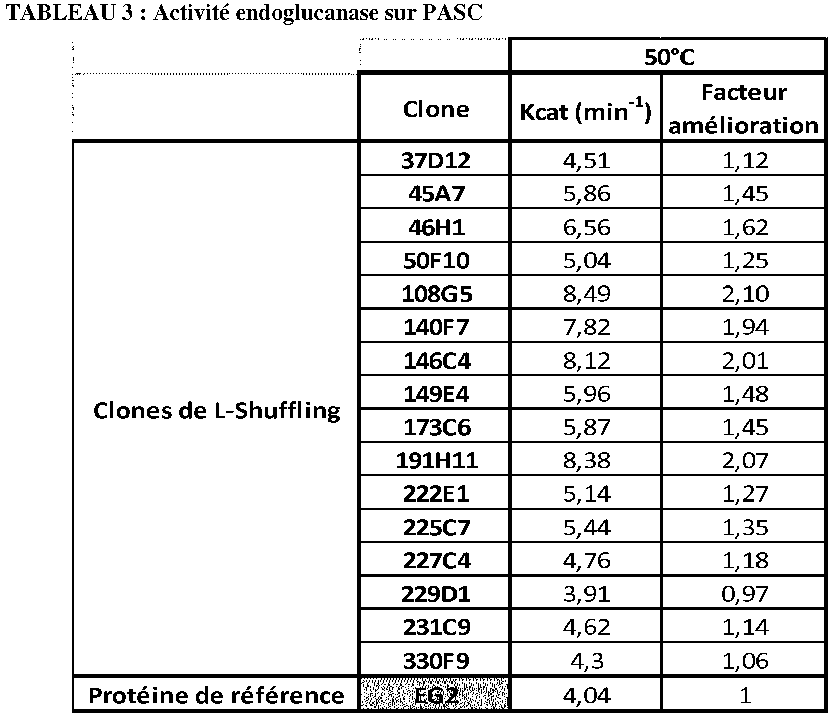

Les résultats montrent des améliorations d'activité enzymatique significatives par rapport à l'enzyme de référence EG2 (SEQ ID NO :2) pour ces clones.

### 2-3/ Sur le substrat Sigmacell

L'amélioration des clones 37D12, 45A7, 46H1, 50F10, 108G5, 140F7, 146C4, 149E4, 173C6, 191H11, 222E1, 225C7, 227C4, 229D1, 231C9 et 330F9 a aussi été évaluée sur un troisième substrat : Sigmacell. Le protocole de test est le même que celui décrit précédemment avec le substrat CMC. L'incubation avec le substrat a lieu pendant 24 h à 50°C.

Le TABLEAU 4 présente la valeur des kcat ainsi que les facteurs d'amélioration obtenus pour les clones 37D12, 45A7, 46H1, 50F10, 108G5, 140F7, 146C4, 149E4, 173C6, 191H11, 222E1, 225C7, 227C4, 229D1, 231C9 et 330F9 à 50°C par rapport à la protéine de référence EG2 (SEQ ID NO :2) dans les conditions expérimentales du test d'activité sur Sigmacell.

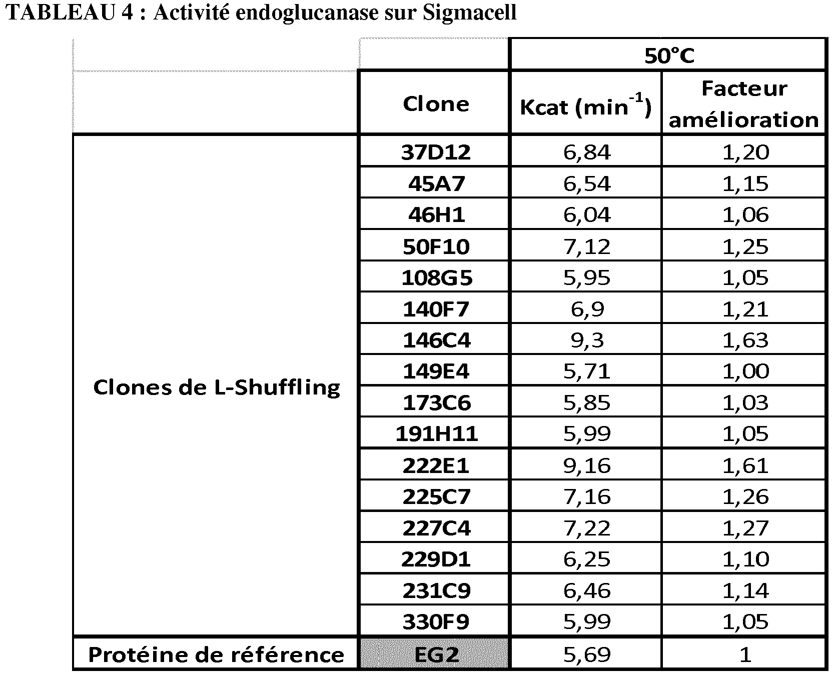

Les résultats montrent que l'amélioration d'activité pour les clones 37D12, 45A7, 46H1, 50F10, 108G5, 140F7, 146C4, 149E4, 173C6, 191H11, 222E1, 225C7, 227C4, 229D1, 231C9 et 330F9 est visible par rapport à l'enzyme de référence EG2 (SEQ ID NO :2)avec le substrat Sigmacell.

### Exemple 2 :

Les variants 146C4, 191H11, 222E1 et 225C7, ainsi que le gène de référence EG2 de *T*. *reesei* (SEQ ID NO :2) ont été clonés entre le promoteur et le terminateur *cbh1* dans le plasmide pUT1040 contenant un gène de résistance à la phléomycine comme marqueur, à l'aide d'une double digestion BamH1/XhoI. 5 µg de chaque vecteur ont été utilisés pour la transformation de la souche *T. reesei* CL847AEG1. Des protoplastes ont été transformés selon une méthode classique connue de l'homme de l'art, par choc calcique et PEG, avec 5 µg de chaque construction. Les transformants ont été sélectionnés sur milieu sélectif PDA/saccharose contenant 30 µg/L de phléomycine. Après trois repiquages successifs permettant l'obtention de clones purs, entre onze et quinze clones ont été obtenus pour chacun des variants. L'ensemble des clones ont été mis en culture dans du milieu F45 (800 µL H₃PO₄ 85%, 4,2 g (NH₄)₂SO₄,0,3 g MgSO₄.7H₂O, 0,75 g CornSteep, 1 mL Oligo Ferment, 6 g potassium phtalate, pH 5,8 - 6) avec 5 g/L glucose et 10 g/L sorbose comme substrat de carbone et inducteur. Après 7 jours de culture à 30°C, le surnageant est prélevé et l'équivalent de 10 mg/L de protéines mesurées par la méthode de Lowry est utilisé pour un test d'activité sur carboxyméthylcellulose.

Pour les mesures d'activité, 150 µL d'une solution CMC 2% dans du tampon citrate 50 mM pH 4,8 sont mélangés avec 150 µL de tampon citrate contenant 10 mg/L de protéines. La réaction est incubée à 50°C et 35°C pendant 10 min, puis inactivée au bain-marie bouillant. Après centrifugation pendant 5 minutes, 20 µL sont prélevés pour réaliser un dosage de sucres réducteurs à l'aide de l'acide 3,5-dinitrosalicylique (DNS). La réduction du DNS et la formation de l'acide 3-amino-5-nitrosalicylique est suivi par lecture de l'absorption à 540 nm, et les sucres réducteurs quantifiés à l'aide d'une gamme de glucose.

Le TABLEAU 5 résume les activités (exprimées en µmol équivalent glucose/mg de protéine/min) obtenues pour les meilleurs clones pour chaque variant. La valeur pour la souche ΔEG1 transformée avec le gène EG2 natif (ΔEG1cEG2) est une moyenne des quatre meilleurs clones.

**TABLEAU 5 : Activité endoglucanase sur CMC**

| **clone** | **activité spécifique 50°C (µmol/mg/min)** | **rapport variant/AEG2cEG2 50°C** | **activité spécifique 35°C (µmol/mg/min)** | **rapport variant/ΔEG2cEG2 35°C** |
|---|---|---|---|---|
| **ΔEG2cEG2** | 10,8 ± 1,8 | | 8,6 ± 2,2 | |
| **191H11/2** | 23,1 ± 1,8 | 2,1 | 9,9 ± 2,8 | 1,2 |
| **191H11/9** | 21,9 ± 4,0 | 2,0 | 10,8 ± 1,6 | 1,3 |
| **191H11/12** | 17,4 ± 0,4 | 1,6 | 9,7 ± 2,0 | 1,1 |
| **146C4/1** | 12,0 ± 1,1 | 1,1 | 8,5 ± 0,7 | 1,0 |
| **146C4/6** | 11,7 ± 0,4 | 1,1 | 8,6 ± 1,2 | 1,0 |
| **146C4/7** | 16,1 ± 1,1 | 1,5 | 11,9 ± 3,2 | 1,4 |
| **222E1/1** | 21,4 ± 0,8 | 2,0 | 13,9 ± 3,3 | 1,6 |
| **222E1/2** | 18,3 ± 1,3 | 1,7 | 13,7 ± 1,4 | 1,6 |
| **222E1/4** | 13,5 ± 0,6 | 1,3 | 10,4 ± 0,8 | 1,2 |
| **222E1/7** | 16,2 ± 1,3 | 1,5 | 11,4 ± 0,3 | 1,3 |
| **225C7/7** | 11,4 ± 0,1 | 1,1 | 10,3 ± 3,3 | 1,2 |
| **225C7/9** | 14,7 ± 0,4 | 1,4 | 8,1 ± 1,4 | 0,9 |

Pour chaque variant, au moins un clone possède une activité CMCase supérieure à la souche ΔEG1cEG2 à 35°C ou à 50°C, les meilleurs clones montrant deux fois plus d'activité que la souche ΔEG1cEG2.

### Exemple 3 : Expression recombinante de l'endoglucanase de référence EG2 et des variants 222E1 et 225C7 chez Saccharomyces cerevisiae

### 1- Production de la protéine de référence l'endoglucanase de référence EG2 et son variant 222E1 dans le milieu extracellulaire

Le gène de l'endoglucanase de référence EG2 de *Trichoderma reesei* (SEQ ID NO :1) et les gènes des variants 222E1 et 225C7 (respectivement SEQ ID NO :23 et 25) ont été clonés sans leur peptide signal dans le vecteur pESC-LeuαAmyc (CNRS-CERMAV). Cette construction permet l'expression des protéines dans le milieu de culture de la souche *Saccharomyces cerevisiae* EBY100, auxotrophe pour la leucine et le tryptophane (Boder ET et Wittrup KD, Biotechnol Prog, 1998, 14:55-62). Ce plasmide permet de placer l'expression des gènes sous le contrôle du promoteur GAL1 inductible au galactose, et possède le gène marqueur de sélection de l'auxotrophie (Leu2) qui permet la sélection des transformants.

La transformation de *Saccharomyces cerevisiae* EBY100 a été réalisée selon les méthodes classiques connues de l'homme de l'art (transformation de levures par choc thermique et acétate de lithium). Les transformants ont été sélectionnés sur milieu YNB 0,67%-Glc 2%-Trp 0,01%.

Un transformant pour chaque gène (Scα-EG2, Scα-222E1 et Scα-225C7) a été utilisé pour ensemencer 15 mL d'un milieu minimum YNB 0,67%-Glc 2%-SD-Trp 0,01%. SD est un mélange d'acides aminés (40 mg/L d'adénine sulfate; 20 mg/L de L-arginine; 100 mg/L de L-acide aspartique; 100 mg/L de L-acide glutamique; 20 mg/L de L-histidine; 30 mg/L de L-lysine; 20 mg/L de L-méthionine; 50 mg/L de L-phénylalanine; 375 mg/L de L-sérine; 200 mg/L de L-thréonine; 30 mg/L de L-tyrosine; 150 mg/L de L-valine et 20 mg/L d'uracile). Après 24 h de préculture à 30°C sous agitation à 220 rpm, les trois souches Scα-EG2, Scα-222E1et Scα-225C7 ont été utilisées pour ensemencer (DO₆₀₀ de 0,5) 150 mL de milieu YNB 0,67%-Gal 2%-SD-Trp 0,01%. Les cultures ont été incubées à 25°C sous agitation à 220 rpm. Après 8 h d'incubation, 6 mL de citrate de sodium à pH 5,6 ont été ajoutés à chaque culture afin de stabiliser le pH à 5.

Après 4 jours d'incubation, 20 mL de culture ont été prélevés. Le surnageant de culture a été obtenu après centrifugation à 3000 g, à 4°C pendant 5 min.

### 2- Détermination de l'activité endoglucanase sur p-nitronhénvl-β-cellotrioside

L'activité endoglucanase des surnageants de culture a été mesurée par hydrolyse du substrat pNPC3 dans un volume de 450 µL dans les conditions suivantes:
- 50 mM de tampon citrate à pH 5
- 2 mM de pNPC3
- 56,3 µL de surnageant de culture des souches Scα-EG2, Scα-221E1 et Scα-225C7
- Incubation à 35°C pendant 6 h

La réaction a été arrêtée en ajoutant 100 µL de carbonate de sodium 1M à 100 µL de réaction d'hydrolyse. La concentration en para-nitrophénol (pNP) libéré par hydrolyse du pNPC3 a été déterminée par mesure de l'absorbance à 415 nm par comparaison avec une gamme étalon de para-nitrophénol (linéaire de 0,36 µM à 360 µM).

Les résultats de la FIGURE 1 montrent que la souche Scα-222E1 possède une activité endoglucanase améliorée d'un facteur 1,6 par rapport à la souche Scα-EG2 qui exprime l'enzyme de référence EG2 (SEQ ID NO :2). La souche Scα-225C7 se révèle, quant à elle, moins efficace pour l'hydrolyse de ce substrat.

### 3- Détermination de l'activité endoglucanase sur carboxyméthylcellulose

L'activité endoglucanase des surnageants de culture a été mesurée par hydrolyse de la carboxyméthylcellulose (CMC) dans un volume de 700 µL dans les conditions suivantes:
- 50 mM de tampon citrate à pH 5
- 1% de CMC
- 210 µL de surnageant de culture des souches Scα-EG2, Scα-222E1et Scα-225C7 dialysés contre du tampon citrate 50 mM pH 5 sur membrane de 10 kDa et concentrés deux fois
- Incubation à 35°C pendant 24 h.

La réaction a été arrêtée en ajoutant 150 µL de réactif au DNS à 100 µL de réaction d'hydrolyse. Après chauffage pendant 5 minutes à 100°C et refroidissement dans la glace, la quantité de sucres réducteurs libérés a été déterminée par mesure de l'absorbance à 550 nm par comparaison avec une gamme étalon réalisée avec du glucose.

Les résultats de la FIGURE 2 indiquent que la quantité de sucres réducteurs libérés par l'action des variants des souches Scα-222E1 et Scα-225C7 est plus importante qu'avec la souche Scα-EG2. Ainsi, après 1 h d'hydrolyse de la CMC, le facteur d'amélioration à 35°C sur ce substrat est de 3,5 pour Scα-222E1 et 2,6 pour Scα-225C7 par rapport à Scα-EG2. Au-delà de ce temps d'incubation, la CMC continue d'être hydrolysée par les deux variants alors que la vitesse de réaction devient pratiquement nulle en présence de la protéine de référence EG2 (SEQ ID NO :2).

### Exemple 4 : Production d'enzymes par T. reesei en fioles alimentées

Les souches de référence et celles présentant la meilleure activité sur CMC (CL847, AEG1, ΔEG1cEG2, 146C4/7, 191H11/9, 222E1/1, 222E1/2, 222E1/7, 225C7/7) ont été cultivées en fioles Erlenmeyer de 250 mL. 55 mL de milieu F45 (10 g/L de tampon dipotassium phtalate pH 6, 4.2g/L (NH₄)₂SO₄, 300 mg/L MgSO₄.7H₂O, 150 mg/L CaCl₂.2H₂O, 1,5 g/L cornsteep, 0,07 % acide orthophosphorique, 5 mg/L FeSO₄, 1,4 mg/L MnSO₄, 1,4 mg/L ZnSO₄,3,7 mg/L CoCl₂ et 12,5 g/L glucose) sont ensemencés et agités à 150 rpm et 30°C. La production se fait en deux phases : une phase batch sur glucose et une phase fed-batch sur lactose. Des prélèvements réguliers permettent de déterminer le moment où la concentration de glucose passe en dessous de 3 g/L. A ce stade, une alimentation fed-batch à l'aide d'un pousse-seringues (6 voies) est lancée. Les cultures sont alimentées par une solution de 50 g/L lactose et 0,3 % NH₃ à un débit de 40 mg de sucre/g de biomasse par heure. Des prélèvements journaliers sont réalisés pour déterminer le pH, le poids sec et la concentration de protéines dans le surnageant. Après 5 jours de culture en fed-batch, la culture est filtrée sur un filtre de 0,45 µm et le surnageant congelé.

La concentration finale de protéines a été de l'ordre de 3 à 4 g/L. Si la concentration était inférieure à 3 g/L, les surnageants ont été concentrés sur colonne (Vivaspin MWCO5 Sartorius).

### Exemple 5: Efficacité des enzymes issus du L-shuffling en hydrolyse de biomasse lignocellulosique selon un procédé SHF

Le substrat de référence utilisé est une paille de blé ayant subi un prétraitement par explosion à la vapeur (19 bars - 3 minutes) après imprégnation acide à 0,01 % H₂SO₄ pendant 10 heures, lavée, neutralisée à pH 5, pressée, séchée. Ses caractéristiques sont présentées en Tableau 9.

**Tableau 6 : Composition de la paille utilisée pour les essais d'hydrolyse**

| **Composition** | **% m/m** |
|---|---|
| WIS | 97,52 |
| Teneur en cendres | 5 |
| Cellulose | 51,7 |
| Xylanes corrigés | 3,57 |
| Hémicellulose | 4,14 |
| Lignine de Klason (surestimée) | 36,49 |
| Acétyl | 0,6 |

Les hydrolyses ont été réalisées à 10 % de matière sèche m/m soit un équivalent de 5,4 % cellulose m/m.

Le taux de protéines est fixé à 10 mg/g de matière sèche soit environ 19 mg/g de cellulose. La concentration des cocktails enzymatiques a été mesurée par la méthode de Lowry en utilisant la BSA comme référence. Chaque cocktail a été supplémenté en activité β-glucosidase à hauteur de 120±2UI/g de cellulose, en ajoutant de la β-glucosidase SP188 (Novozymes).

Les essais sont effectués en tubes Eppendorf de 2mL utile (1g réactionnel) contenant :
- 0,11 ± 0,001 g de substrat paille lavée
- 0,9 ± 0,02 mL de milieu réactionnel d'hydrolyse composé de tampon acétate 50 mM - pH 4,8 et chloramphénicol (0,05 g/L)
- entre 0,1 et 0,2 ± 0,02 g de cocktail enzymatique en fonction de leur taux de protéines

Les hydrolyses enzymatiques sont réalisées à 45 ± 2°C sous agitation type vortex à 900 rotations par minute dans un *Thermomixer Comfort Eppendorf.*

Tous les essais sont effectués en duplicat avec des temps de prélèvement fixés à t 24, 48 et 96 heures avec pour certains des prélèvements à t 72 heures.

A chaque temps de prélèvement, les hydrolysats sont ébouillantés 5 minutes dans les tubes Eppendorf sacrifiés. Ces tubes sont ensuite refroidis et centrifugés. Le dosage du glucose est effectué par HPLC. Parallèlement les résidus solides de chaque tube eppendorf sont lavés et centrifugés 3 fois avant d'être séchés à 105°C pendant 24 heures de façon à évaluer les WIS (Water Insoluble Solids). Le calcul du rendement d'hydrolyse est effectué en tenant compte des WIS.

Les cocktails issus de l'exemple 4 ont été évalués. Deux tests témoins sont effectués avec les cocktails de référence supplémentés également en β-glucosidase pour comparaison : un cocktail produit par la souche CL847 AEG1 (ΔEG1) et un cocktail produit par la souche CL847 ΔEG1 retransformée avec le gène de référence EG2 (ΔEG1cEG2).

La FIGURE 3 présente les résultats de SHF pour le cocktail issu de la souche 146C4/7 (SEQ ID NO :16).

Les résultats présentés en FIGURE 3 montrent que la vitesse initiale d'hydrolyse du cocktail produit par le variant 146C4 est supérieure à celles des cocktails de référence ΔEG1 et AEG1cEG2. Le rendement final d'hydrolyse est également supérieur à celui des cocktails de référence ΔEG1 et AEG1cEG2.

La FIGURE 4 présente les résultats de SHF pour le cocktail issu de la souche 222E1/1 (SEQ ID NO :24) et le cocktail issu de la souche 225C7/7 (SEQ ID NO :26).

Les résultats présentés en FIGURE 4 montrent que les vitesses initiales d'hydrolyse des cocktails produits par le variant 222E1 et le variant 225C7 sont supérieures à celles des cocktails de référence ΔEG1 et AEG1cEG2. Les rendements finaux d'hydrolyse sont également supérieurs à ceux des cocktails de référence ΔEG1 et AEG1cEG2.

### Exemple 6 : Efficacité des enzymes en hydrolyse de biomasse lignocellulosique selon un procédé SSF

Le substrat utilisé est le même que celui décrit en TABLEAU 6 (Exemple 4).

Les SSF sont réalisées en triplicat dans des réacteurs de laboratoire. Ils sont constitués des éléments suivants :
- un flacon en verre de 30 mL de volume utile ;
- un bouchon de sécurité en polyétheréthercétone (Peek) ;
- une soupape unidirectionnelle DV-118 commercialisée par la société Vaplock fixée à travers le bouchon. La soupape est configurée pour s'ouvrir en sortie lorsque la pression relative dans le flacon est supérieure à 70 mbar ;
- un tube creux en polypropylène, emmanché à travers un second traversant le bouchon et équipé au niveau de l'extrémité inférieure dudit tube d'un septum ;
- un joint plat disposé entre le goulot du flacon et le bouchon.

Le principe de mise en oeuvre des bioréacteurs est le suivant : le CO₂ produit lors de la fermentation éthanolique s'accumule dans le ciel situé au-dessus du milieu réactionnel entrainant par accumulation une augmentation de la pression dans le bioréacteur (P_{G}). Lorsque P_{G} devient supérieure à la pression d'ouverture de la soupape unidirectionnelle (P_{S}), celle-ci s'ouvre pour laisser échapper une quantité de gaz qui est par exemple déterminée par pesée. Lorsque P_{G} < P_{S}, la soupape se referme jusqu'à ce que P_{G} soit supérieur à P_{S}. Ainsi le bioréacteur en fonctionnement est toujours sous pression de manière à assurer un milieu anaérobie stable pour la fermentation. La quantité d'éthanol produite est évaluée par la production de CO₂ estimée par perte de poids à partir de l'équation stoechiométrique de fermentation du glucose en éthanol suivante :

C₆H₁₂O₆ (glucose) → 2 CO₂ + 2 CH₃CH₂OH (éthanol) + énergie

Le milieu de culture utilisé pour la SSF est un milieu aqueux qui comprend :
- un tampon acétate 50 mM pour pH 5 ;
- du chloramphénicol à 0,1 g/L ;
- du milieu nutritif à 3 g/L de KH₂PO₄, 2 g/L de (NH₄)₂SO₄, 0,4 g/L de MgSO₄,7H₂O et 1 g/L d'extrait de levure.

Les SSF ont été réalisées à 10±0.01% m/m de matière sèche soit un équivalent de 5,4 % cellulose m/m pour une masse réactionnelle totale de 15 ± 0,003 g. Le taux de protéines est fixé à 10 ± 0,01 mg de cellulases par gramme de matière sèche, soit environ 19 mg/g cellulose. La concentration des cocktails enzymatiques a été mesurée par la méthode de Lowry en utilisant la BSA (Bovine Serum Albumine) comme référence. Chaque cocktail a été supplémenté en activité β-glucosidase à hauteur de 120±2 UI/g cellulose, en ajoutant de la β-glucosidase SP188 (Novozymes).

La levure de fermentation des sucres *(Saccharomyces cerevisiae,* souche Ethanol Red, Fermentis, France) est ajoutée dans le milieu pour obtenir une teneur de 2±0,1 g/kg.

Les enzymes et les levures sont ajoutées dans les bioréacteurs après une heure de conditionnement de la paille de blé prétraitée à 35°C avec le tampon, le chloramphénicol et le milieu de culture.

La réaction de SSF est conduite à une température d'environ 35°C, en plaçant le bioréacteur de laboratoire dans un incubateur de type INFORS Multitron HT Standard avec une vitesse de rotation orbitale de 150 tours par minute.

Au cours du temps, la perte de masse a été suivie par pesée des bioréacteurs. A la fin de la réaction, le moût de fermentation est chauffé à 100°C pendant 5 minutes, refroidi et centrifugé pour séparer les matières solides non hydrolysées du jus de fermentation. Ce dernier est ensuite analysé par chromatographie en phase gazeuse afin de déterminer sa concentration en éthanol.

Les cocktails issus de l'exemple 4 ont été évalués. Deux tests témoins sont effectués avec les cocktails de référence supplémentés également en β-glucosidase pour comparaison : un produit par la souche CL847 AEG1 (ΔEG1) et un par la souche CL847 AEG1 retransformée avec le gène de référence EG2 (ΔEG1cEG2).

La FIGURE 5 présente les résultats de SSF pour le cocktail issu de la souche 146C4/7 et pour le cocktail issu de la souche 191H11/9 (SEQ ID NO 22):
Les résultats présentés en FIGURE 5 montrent que les avancements (productions d'éthanol pour la même dose d'enzymes) de la SSF sur 100 heures pour le cocktail exprimant l'endoglucanase 146C4 et le cocktail exprimant l'endoglucanase 191H11 sont supérieurs à ceux des cocktails de référence ΔEG1 et ΔEG1cEG2.

La FIGURE 6 présente les résultats de SSF pour les 3 cocktails issus des souches 222E1/1, 222E1/2 et 222E1/7 (moyenne des résultats obtenus avec les 2 variants) et pour le cocktail issu de la souche 225C7/7.

Les résultats présentés en FIGURE 6 montrent que les avancements de la SSF sur 100 heures pour le cocktail exprimant l'endoglucanase 222E1 et le cocktail exprimant l'endoglucanase 225C7 sont équivalents et supérieurs à ceux des cocktails de référence ΔEG2 et ΔEG1cEG2.

## Revendications

1. Polypeptide isolé ou purifié **caractérisé en ce qu'**il a une activité endoglucanase améliorée par rapport à l'activité endoglucanase de la protéine de référence EG2, ledit polypeptide étant choisi dans le groupe consistant en :
i) une séquence d'acides aminés choisie parmi SEQ ID NO :4, , SEQ ID NO :8, SEQ ID NO :10, SEQ ID NO :12; SEQ ID NO :14, SEQ ID NO :16, SEQ ID NO :18, SEQ ID NO :20, SEQ ID NO :22, SEQ ID NO :24, SEQ ID NO :26, SEQ ID NO :28, SEQ ID NO :30, SEQ ID NO :32 et SEQ ID NO :34;
ii) une séquence d'acides aminés présentant un pourcentage d'identité d'au moins 98%, préférentiellement 99%, par rapport à la séquence SEQ ID NO :4, SEQ ID NO :14, SEQ ID NO :18, SEQ ID NO :20, SEQ ID NO :22, SEQ ID NO :24, SEQ ID NO :26, SEQ ID NO :28, SEQ ID NO :30, SEQ ID NO :32 ou SEQ ID NO :34.

2. Acide nucléique purifié ou isolé, **caractérisé en ce qu'**il code au moins un polypeptide selon la revendication **1.**

3. Acide nucléique purifié ou isolé selon la revendication 2 choisi parmi les séquences suivantes: SEQ ID NO :3, SEQ ID NO :7, SEQ ID NO :9, SEQ ID NO :11; SEQ ID NO :13, SEQ ID NO :15, SEQ ID NO :17, SEQ ID NO :19, SEQ ID NO :21, SEQ ID NO :23, SEQ ID NO :25, SEQ ID NO :27, SEQ ID NO :29, SEQ ID NO :31 et SEQ ID NO :33.

4. Vecteur **caractérisé en ce qu'**il comprend un acide nucléique selon l'une des revendications 2 ou 3.

5. Cellule hôte isolée **caractérisée en ce qu'**elle comprend l'acide nucléique selon l'une des revendications **2 ou 3** ou le vecteur selon la revendication **4.**

6. Cellule hôte isolée selon la revendication **5, caractérisée en ce qu'**elle est choisie parmi *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia* et *Saccharomyces.*

7. Cellule hôte isolée selon la revendication **5 ou 6, caractérisée en ce qu'**elle est choisie parmi *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* et *Saccharomyces cerevisiae.*

8. Utilisation dudit polypeptide selon la revendication **1** pour l'hydrolyse de la cellulose.

9. Utilisation dudit polypeptide selon la revendication **1** pour la production de biocarburant.

10. Composition enzymatique apte à agir sur la biomasse lignocellulosique comprenant au moins un polypeptide selon la revendication **1.**

11. Procédé de production de biocarburant à partir de la biomasse, **caractérisé en ce qu'**il comprend les étapes successives suivantes :
- mise en suspension en phase aqueuse de la biomasse à hydrolyser;
- hydrolyse en présence d'une composition enzymatique selon la revendication **10** de la biomasse lignocellulosique de manière à produire un hydrolysat contenant du glucose ;
- fermentation du glucose de l'hydrolysat de manière à produire un moût de fermentation ;
- séparation du biocarburant du moût de fermentation.

12. Procédé de production de biocarburant à partir de la biomasse, **caractérisé en ce qu'**il comprend les étapes successives suivantes :
- mise en suspension en phase aqueuse de la biomasse à hydrolyser ;
- ajout simultané d'une composition enzymatique selon la revendication **10** et d'un organisme fermentaire de manière à produire un moût de fermentation ;
- séparation du biocarburant du moût de fermentation.

13. Procédé selon la revendication 12, dans lequel l'organisme fermentaire est choisi parmi une cellule hôte selon l'une des revendications 6 ou 7.

14. Procédé de production de biocarburant à partir de la biomasse, **caractérisé en ce qu'**il comprend les étapes successives suivantes :
- mise en suspension en phase aqueuse de la biomasse à hydrolyser ;
- ajout d'une ou plusieurs cellules hôtes selon l'une des revendications **5 à 7** avec un organisme fermentaire et/ou une composition enzymatique selon la revendication 10, de manière à produire un moût de fermentation ;
- séparation du biocarburant du moût de fermentation.

## Patentansprüche

1. Isoliertes oder gereinigtes Polypeptid, **dadurch gekennzeichnet, dass** es eine verbesserte Endoglucanase-Aktivität aufweist im Vergleich zur Endoglucanase-Aktivität des Referenzproteins EG2, wobei das Polypeptid gewählt ist aus der Gruppe, umfassend:
i) eine Aminosäuresequenz, die gewählt ist aus SEQ ID NO: 4, , SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32 und SEQ ID NO:34;
ii) eine Aminosäuresequenz, die im Vergleich zu der Sequenz SEQ ID NO: 4, SEQ ID NO: 14, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32 oder SEQ ID NO: 34 eine Sequenzidentität von wenigstens 98%, bevorzugt 99% aufweist.

2. Gereinigte oder isolierte Nukleinsäure, **dadurch gekennzeichnet, dass** sie wenigstens ein Polypeptid nach Anspruch **1** kodiert.

3. Gereinigte oder isolierte Nukleinsäure nach Anspruch 2, welche gewählt ist aus den folgenden Sequenzen: SEQ ID NO: 3, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO:23, SEQ ID NO: 25, SEQ ID NO: 27, SEQ ID NO: 29, SEQ ID NO: 31 und SEQ ID NO: 33.

4. Vektor, **dadurch gekennzeichnet, dass** er eine Nukleinsäure nach einem der Ansprüche 2 oder 3 umfasst.

5. Isolierte Wirtszelle, **dadurch gekennzeichnet, dass** sie die Nukleinsäure nach einem der Ansprüche **2 oder 3** oder den Vektor nach Anspruch **4** umfasst.

6. Isolierte Wirtszelle nach Anspruch **5, dadurch gekennzeichnet, dass** sie gewählt ist aus *Trichoderma, Aspergillus, Neurospora, Humicola, Penicillium, Fusarium, Thermomonospora, Myceliophthora, Chrysosporium, Bacillus, Pseudomonas, Escherichia, Cdostridium, Cellulomonas, Streptomyces, Yarrowia, Pichia* und *Saccharomyces.*

7. Isolierte Wirtszelle nach Anspruch **5 oder 6,**
**dadurch gekennzeichnet, dass** sie gewählt ist aus *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopile, Chrysosporium lucknowense, Penicillium pinophidum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* und *Saccharomyces cerevisiae.*

8. Verwendung des Polypeptids nach Anspruch 1 zur Hydrolyse der Cellulose.

9. Verwendung des Polypeptids nach Anspruch **1** zur Herstellung von Biokraftstoff.

10. Enzymatische Zusammensetzung, welche in der Lage ist, auf lignocellulosehaltige Biomasse zu wirken und wenigstens ein Polypeptid nach Anspruch **1** umfasst.

11. Herstellungsverfahren für Biokraftstoff ausgehend von Biomasse, **dadurch gekennzeichnet, dass** es die folgenden aufeinanderfolgenden Schritte umfasst:
- Suspendieren der zu hydrolysierenden Biomasse in wässriger Phase;
- Hydrolyse der lignocellulosehaltigen Biomasse in Anwesenheit einer enzymatischen Zusammensetzung nach Anspruch **10** zur Herstellung eines glucosehaltigen Hydrolysats;
- Fermentation der Glucose des Hydrolysats zur Herstellung eines Fermentationsmosts;
- Trennen des Biokraftstoffs vom Fermentationsmost.

12. Herstellungsverfahren für Biokraftstoff ausgehend von Biomasse, **dadurch gekennzeichnet, dass** es die folgenden aufeinanderfolgenden Schritte umfasst:
- Suspendieren der zu hydrolysierenden Biomasse in wässriger Phase;
- gleichzeitiges Hinzugeben einer enzymatischen Zusammensetzung nach Anspruch **10** und eines Fermentierungsorganismus zur Herstellung eines Fermentationsmostes;
- Trennen des Biokraftstoffs vom Fermentationsmost.

13. Verfahren nach Anspruch 12, bei welchem der Fermentierungsorganismus aus einer Wirtszelle nach einem der Ansprüche 6 oder 7 gewählt ist.

14. Herstellungsverfahren für Biokraftstoff ausgehend von Biomasse, **dadurch gekennzeichnet, dass** es die folgenden aufeinanderfolgenden Schritte umfasst:
- Suspendieren der zu hydrolysierenden Biomasse in wässriger Phase;
- Hinzufügen einer oder mehrerer Wirtszellen nach einem der Ansprüche **5 bis 7,** mit einem Fermentierungsorganismus und/oder einer enzymatischen Zusammensetzung nach Anspruch 10 zur Herstellung eines Fermentationsmostes;
- Trennen des Biokraftstoffs vom Fermentationsmost.

## Claims

1. Isolated or purified polypeptide **characterised in that** it has an improved endoglucanase activity with respect to the endoglucanase activity of the reference protein EG2, said polypeptide being chosen from the group consisting of:
i) an amino acid sequence chosen from SEQ ID NO: 4, SEQ ID NO: 8, SEQ ID NO: 10, SEQ ID NO: 12; SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32 and SEQ ID NO: 34;
ii) an amino acid sequence having a percentage identity of at least 98%, preferably 99%, with respect to the sequence SEQ ID NO: 4, SEQ ID NO: 14, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32 or SEQ ID NO: 34.

2. Purified or isolated nucleic acid, **characterised in that** it encodes at least one polypeptide according to claim 1.

3. Purified or isolated nucleic acid according to claim 2 chosen from the following sequences: SEQ ID NO: 3, SEQ ID NO: 7, SEQ ID NO: 9, SEQ ID NO: 11; SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31 and SEQ ID NO: 33.

4. Vector **characterised in that** it comprises a nucleic acid according to one of claims 2 or 3.

5. Isolated host cell **characterised in that** it comprises nucleic acid according to one of claims 2 or 3 or the vector according to claim 4.

6. Isolated host cell according to claim 5, **characterised in that** it is chosen from *Trichoderma, Aspergillus, Neurospora, Humicoda, Penicillium, Fusarium, Thermomonospora, Myceliophthora, Chrysosporium, Bacillus, Pseudomonas, Escherichia, Cdostridium, Cellulomonas, Streptomyces, Yarrowia, Pichia and Saccharomyces.*

7. Isolated host cell according to claim 5 or 6, **characterised in that** it is chosen from *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopile, Chrysosporium lucknowense, Penicillium pinophilum, Penicillium oxalicum, Escherichia code, Clostridium acetobutylicum, Clostridium saccharolyticum, Clostridium benjerinckii, Clostridium butylicum, Pichia pastoris, Yarrowia lipolityca and Saccharomyces cerevisiae.*

8. Use of said polypeptide according to claim 1 for the hydrolysis of cellulose.

9. Use of said polypeptide according to claim 1 for the production of biofuel.

10. Enzymatic composition able to act on the lignocellulosic biomass comprising at least one polypeptide according to claim 1.

11. Method for producing biofuel from biomass, **characterised in that** it comprises the following successive steps:
- putting into suspension in aqueous phase the biomass to be hydrolysed;
- hydrolysis in the presence of an enzymatic composition according to claim 10 of the lignocellulosic biomass in such a way as to produce a hydrolysate containing glucose;
- fermentation of the glucose of the hydrolysate in such a way as to produce a fermentation must;
- separation of the biofuel from the fermentation must.

12. Method for producing biofuel from biomass, **characterised in that** it comprises the following successive steps:
- putting into suspension in aqueous phase the biomass to be hydrolysed;
- simultaneous adding of an enzymatic composition according to claim 10 and of a fermentation organism in such a way as to produce a fermentation must;
- separation of the biofuel from the fermentation must.

13. Method according to claim 12, wherein the fermentation organism is chosen from a host cell according to one of claims 6 or 7.

14. Method for producing biofuel from biomass, **characterised in that** it comprises the following successive steps:
- putting into suspension in aqueous phase of the biomass to be hydrolysed;
- adding of one or several host cells according to one of claims 5 to 7 with a fermentation organism and/or enzymatic composition according to claim 10, in such a way as to produce a fermentation must;
- separation of the biofuel from the fermentation must.
